# EUROPEAN PATENT APPLICATION

(11) **EP 3 909 952 A1**
(43) Date of publication of application: **17.11.2021**
(21) Application number: 20738274.8
(22) Date of filing: 10.01.2020
(51) Int. Cl.: C07D 471/14, A61K 31/405

(54) **SALTS OF HETEROCYCLIC COMPOUND AND USE THEREOF**

(30) Priority: 10.01.2019 CN 201910024238; 22.05.2019 CN 201910431026
(71) Applicant: CSPC Zhongqi Pharmaceutical Technology (Shijiazhuang) Co., Ltd., High-Tech Development Zone Shijiazhuang Hebei 050035 (CN)
(72) Inventor: SUN, Xiaowei, Shijiazhuang, Hebei 050035 (CN); ZHANG, Qianru, Shijiazhuang, Hebei 050035 (CN); LYU, Shuo, Shijiazhuang, Hebei 050035 (CN); ZHANG, Hongfen, Shijiazhuang, Hebei 050035 (CN); GAO, Yuan, Shijiazhuang, Hebei 050035 (CN); YANG, Hanyu, Shijiazhuang, Hebei 050035 (CN); ZHENG, Ligang, Shijiazhuang, Hebei 050035 (CN); LIU, Xibao, Shijiazhuang, Hebei 050035 (CN); WU, Ximei, Shijiazhuang, Hebei 050035 (CN)
(74) Representative: dompatent von Kreisler Selting Werner - Partnerschaft von Patent- und Rechtsanwälten mbB
(86) International application number: PCT/CN2020/071515
(87) International publication number: WO 2020/143793

(57) **Abstract**

Disclosed are a pharmaceutically acceptable salt of a heterocyclic compound depicted by formula (A) or a hydrate of the salt. The pharmaceutically acceptable salt is selected from alkali metal salts. The present inventors have unexpectedly discovered that three salts, especially sodium salt, lithium salt and a hydrate of either salt, have improved solubility and dissolution when compared to the compound of formula A, and thus can be more easily formulated into a drug product when compared to the compound of formula A. Also disclosed is a use of the heterocyclic compound or a hydrate depicted by formula A, a pharmaceutically acceptable alkali metal salt thereof or a hydrate of the alkali metal salt in the preparation of a medicament for treating acute lung injury or acute respiratory distress syndrome. Experimental results indicated that said compound greatly alleviated acute lung injury or acute respiratory distress syndrome induced by cigarette smoke (CS) or lipopolysaccharide (LPS) by inhibiting infiltration of the macrophages and neutrophils in the lung, decreasing pulmonary vascular permeability, reducing the generation of pro-inflammatory cytokines and cytokine/chemokines, and promoting the generation of IL-10.

## Description

This application claims the priorities of Chinese application No. 201910024238.0 with a title of "salts of heterocyclic compounds as CRTH2 receptor antagonists and preparation method and use thereof" filed with China National Intellectual Property Administration on January 10, 2019, and Chinese application No. 201910431026.4 with a title of "use of heterocyclic compounds and salts thereof" filed with China National Intellectual Property Administration on May 22, 2019, and the entire contents of these two applications are incorporated into this application by reference.

### Technical field

The invention belongs to the technical field of medicine, and specifically relates to heterocyclic compound salts and use thereof.

### Background technique

Acute lung injury/acute respiratory distress syndrome (ALI/ARDS) refers to acute and progressive hypoxic respiratory failure caused by various pathogenic factors inside and outside the lung other than cardiogenic factors. Since Ashbaugh et al. reported adult respiratory distress syndrome (ARDS) in 1967, it has attracted great interests of many domestic and foreign scholars and a lot of clinical and experimental research works have been done. ALI/ARDS symposiums were successively held in China, and there was an in-depth discussion mainly on the definition, pathogenesis, diagnostic criteria and treatment of ALI/ARDS. The understanding of ALI/ARDS has been significantly improved. It is manifested in the gradual standardization of the naming and definition of ALI/ARDS, and a deeper understanding of its pathogenesis. The diagnostic criteria of ALI/ARDS convenient for clinical use have been proposed, and some more mature treatment experiences and measures have been accumulated, and the incidence and mortality of ALI/ARDS have a downward trend. However, it must be clearly recognized that the etiology and pathogenesis of ALI/ARDS are complicated, and there are many pathogenic causes. At present, there is still a lack of effective treatment measures for the pathogenesis, and the mortality rate of ALI/ARDS is still high. Therefore, it is necessary to develop drugs for treatment of ALI/ARDS.

CN101896178B discloses a heterocyclic compound represented by the following formula I as CRTH2 receptor antagonist, 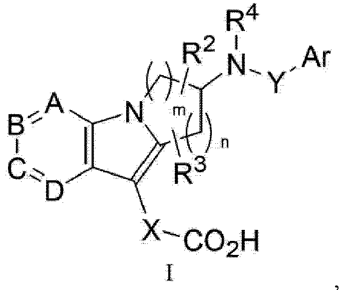 wherein, a compound with the following structure is disclosed in example 3:

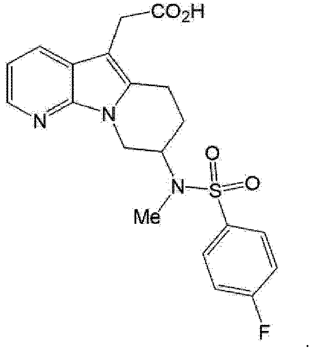

Those skilled in the art know that although it is common for pharmaceutical compounds to have crystal forms, whether a certain pharmaceutical compound can obtain a crystal form often requires a lot of experiments and screening before it is possible to obtain a desired product. Moreover, the development of products with improved effects on this basis has become a further demand for a long time. Therefore, one of the important tasks of drug workers includes the discovery of stable crystal forms of drugs. However, the resolution of these problems is rarely as simple as seen in hindsight. An efficient drug development process must focus on the comprehensive consideration of product quality, repeatability, durability and cost-effectiveness.

### Summary of the invention

The inventors have found that the heterocyclic compound represented by the following formula A is insoluble in water, which seriously affects the pharmaceutical properties of the heterocyclic compound represented by formula A. Therefore, it is necessary to improve its structure to meet the requirements of pharmaceuticals.

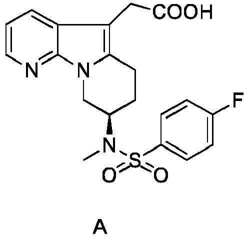

In order to solve the problems above, the present invention provides a pharmaceutically acceptable salt of a heterocyclic compound represented by formula A or a hydrate of the salt, wherein the pharmaceutically acceptable salt is selected from alkali metal salts, preferably sodium salt, lithium salt or potassium salt,

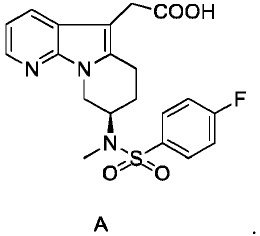

Preferably, the compound of formula A is in the form of a hydrate, wherein the mass fraction of water is 3.5-5.0%.

According to the present invention, the hydrate is preferably a monohydrate.

As an example, the hydrate is selected from a compound represented by the following formula A-N, A-L or A-K:

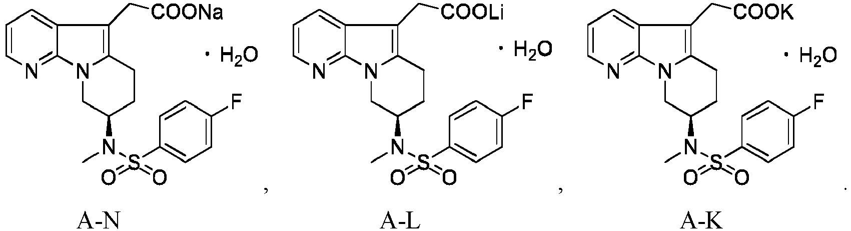

Preferably, the compound represented by formula A-N is a crystalline hydrate, which has characteristic peaks at 2θ angles of 16.4±0.2°, 18.9±0.2°, 21.7±0.2°, and 24.0±0.2° in the X-ray powder diffraction spectrum using Cu-Ka radiation.

Preferably, it has characteristic peaks at 2θ angles of 11.8±0.2°, 16.4±0.2°, 16.7±0.2°, 16.9±0.2°, 17.1±0.2°, 17.8±0.2°, 18.6±0.2°, 18.9±0.2°, 21.7±0.2°, 23.7±0.2°, and 24.0±0.2° in the X-ray powder diffraction spectrum using Cu-Ka radiation.

More preferably, the crystalline hydrate has characteristic peaks at 2θ angles of 5.6±0.2°, 11.8±0.2°, 14.0±0.2°, 15.8±0.2°, 16.4±0.2°, 16.7±0.2°, 16.9±0.2°, 17.1±0.2°, 17.8±0.2°, 18.6±0.2°, 18.9±0.2°, 20.3±0.2°, 21.7±0.2°, 23.7±0.2°, 24.0±0.2°, 26.1±0.2°, 28.1±0.2°, 28.5±0.2°, and 29.8±0.2° in the X-ray powder diffraction spectrum using Cu-Ka radiation.

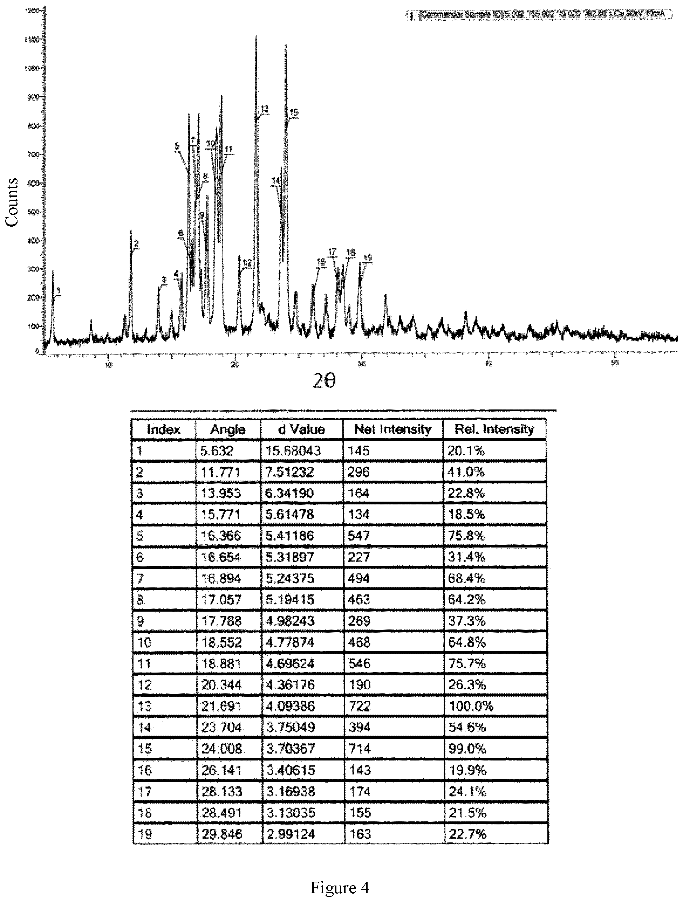

Further preferably, the crystalline hydrate has an X-ray powder diffraction spectrum (XRPD) substantially as shown in FIG. 4.

Preferably, the mass fraction of water in the crystalline hydrate of the sodium salt is 3.4-4.4%, and more preferably 3.6-4.2%.

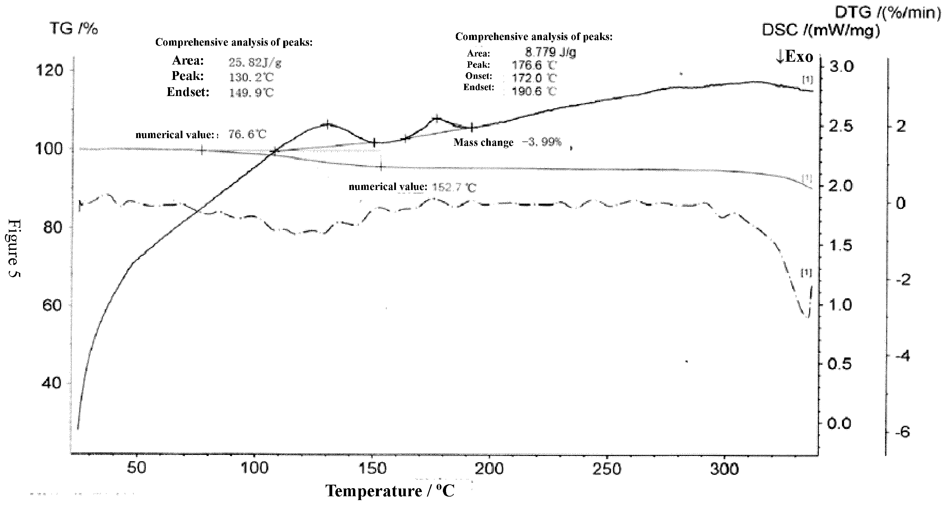

More preferably, the crystalline hydrate of the sodium salt has a DSC-TGA spectrum substantially as shown in FIG. 5.

Preferably, the mass fraction of water in the crystalline hydrate of the sodium salt is 3.5-4.5%, and more preferably 3.9-4.3%.

According to the present invention, the potassium salt compound represented by formula A-K is a crystalline hydrate, which has characteristic peaks at 2θ angles of 15.6±0.2°, 21.4±0.2°, and 24.0±0.2° in the X-ray powder diffraction spectrum using Cu-Ka radiation.

Preferably, the potassium salt compound represented by formula A-K is a crystalline hydrate, which has characteristic peaks at 2θ angles of 11.7±0.2°, 15.6±0.2°, 16.6±0.2°, 17.9±0.2°, 18.5±0.2°, 21.4±0.2°, 24.0±0.2°, and 28.2±0.2° in the X-ray powder diffraction spectrum using Cu-Ka radiation.

Preferably, the potassium salt compound represented by formula A-K is a crystalline hydrate, which has characteristic peaks at 2θ angles of 11.7±0.2°, 15.6±0.2°, 15.9±0.2°, 16.6±0.2°, 17.4±0.2°, 17.9±0.2°, 18.5±0.2°, 21.4±0.2°, 23.5±0.2°, 24.0±0.2°, 27.7±0.2°, and 28.2±0.2° in the X-ray powder diffraction spectrum using Cu-Ka radiation.

More preferably, the crystalline hydrate of the potassium salt has characteristic peaks at 2θ angles of 11.7±0.2°, 14.0±0.2°, 15.6±0.2°, 15.9±0.2°, 16.6±0.2°, 17.4±0.2°, 17.9±0.2°, 18.5±0.2°, 20.1±0.2°, 21.4±0.2°, 23.5±0.2°, 24.0±0.2°, 27.5±0.2°, 27.7±0.2°, 28.2±0.2°, 28.6±0.2°, 29.3±0.2°, and 29.6±0.2° in the X-ray powder diffraction spectrum using Cu-Ka radiation.

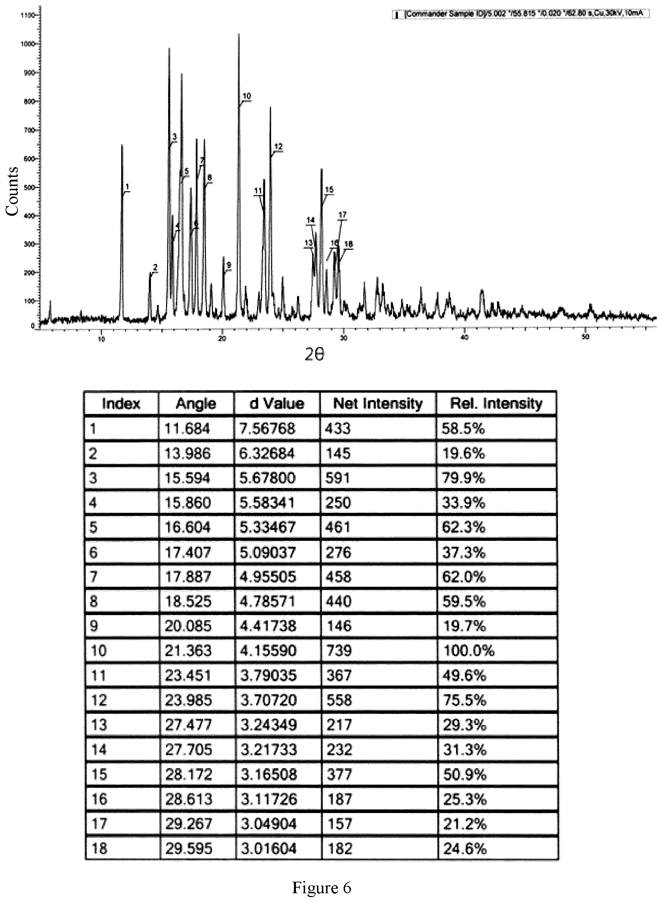

Further preferably, the crystalline hydrate of the potassium salt has an X-ray powder diffraction spectrum substantially as shown in FIG. 6.

Preferably, the mass fraction of water in the crystalline hydrate of the potassium salt is 3.3-4.3%, and more preferably 3.5-4.1%.

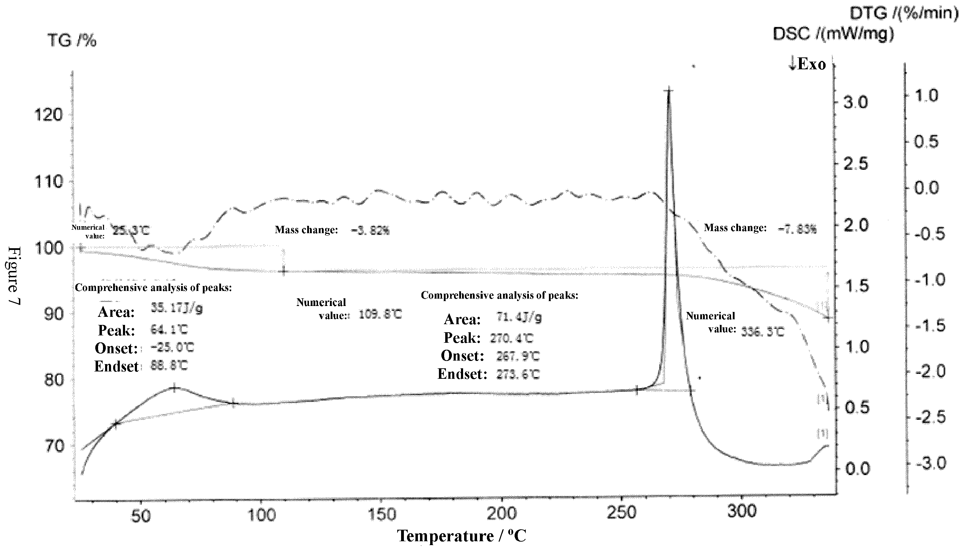

Further preferably, the crystalline hydrate of the potassium salt has a DSC-TGA spectrum substantially as shown in FIG. 7.

According to the present invention, the lithium salt compound represented by formula A-L is a crystalline hydrate, which has characteristic peaks at 2θ angles of 16.7±0.2°, 18.8±0.2°, 21.9±0.2°, and 23.9±0.2° in the X-ray powder diffraction spectrum using Cu-Ka radiation.

More preferably, the crystalline hydrate of the lithium salt has characteristic peaks at 2θ angles of 5.6±0.2°, 8.8±0.2°, 11.8±0.2°, 14.0±0.2°, 16.3±0.2°, 16.7±0.2°, 16.9±0.2°, 17.0±0.2°, 17.7±0.2°, 18.5±0.2°, 18.8±0.2°, 21.9±0.2°, 23.9±0.2°, and 28.2±0.2° in the X-ray powder diffraction spectrum using Cu-Ka radiation.

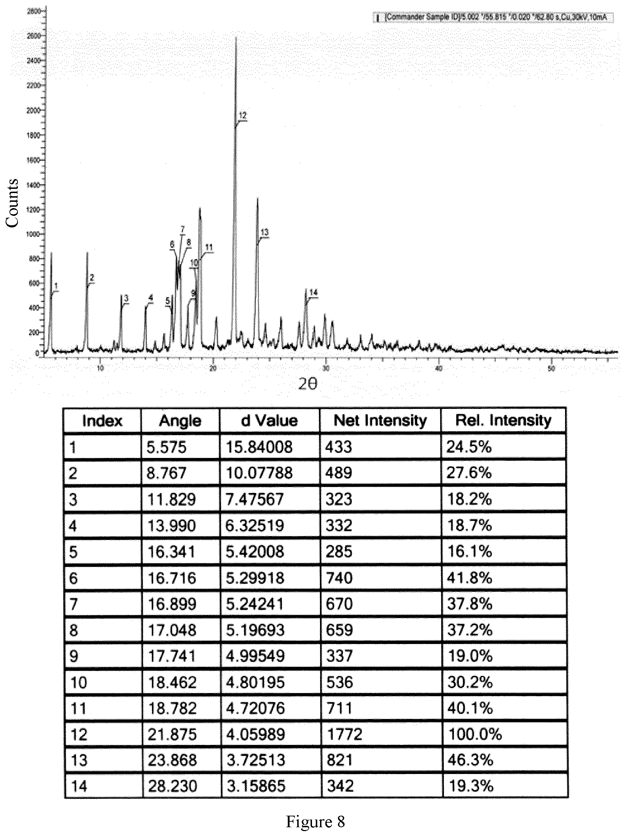

Further preferably, the crystalline hydrate of the lithium salt has an X-ray powder diffraction spectrum substantially as shown in FIG. 8.

Preferably, the mass fraction of water in the crystalline hydrate of the lithium salt is 3.6-4.6%, and more preferably 3.9-4.5%.

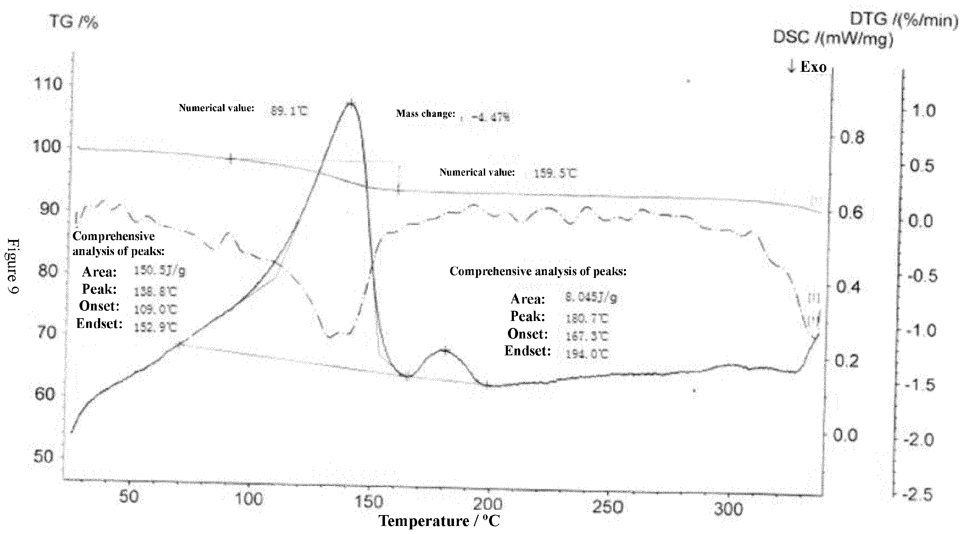

More preferably, the crystalline hydrate of the lithium salt has a DSC-TGA spectrum substantially as shown in FIG. 9.

The present invention also provides a method for preparing a pharmaceutically acceptable salt of the heterocyclic compound represented by formula A or a hydrate of the salt, which comprises the following steps:
dissolving the compound represented by formula A in a ketone solvent; adding an aqueous solution of alkali metal hydroxide to the ketone solvent for reaction; and then filtering and drying to obtain it; wherein the alkali metal hydroxide is preferably sodium hydroxide, lithium hydroxide or potassium hydroxide.

The heterocyclic compound represented by formula A in the present invention can be prepared by referring to the methods described in Examples 3 and 4 in CN101896178B. For example, the racemate of the heterocyclic compound represented by formula A is eluted on a Chiralcel OJ-RH column (Chiralcel Technologies) with a methanol solution containing 0.05% trifluoroacetic acid to separate the heterocyclic compound represented by formula A.

According to the preparation method of the present invention, the concentration of the aqueous solution of alkali metal hydroxide is (0.1-1) g/mL, preferably (0.15-0.55) g/mL, for example, 0.153, 0.375 or 0.533 g/mL.

According to the preparation method of the present invention, the ketone solvent is selected from acetone or methyl ethyl ketone.

According to the preparation method of the present invention, the volume ratio of the ketone solvent to the aqueous solution of the alkali metal hydroxide is (40-60):1, for example, 50:1 or 52.5:1.

The pharmaceutically acceptable salt of the heterocyclic compound represented by formula A or the hydrate of the salt provided by the present invention has the ability to interact with prostaglandin receptors so that it can be used to prevent or reverse adverse symptoms caused by prostaglandin in mammals, especially humans. This simulation or antagonism of prostaglandin shows that the compounds of the present invention and their pharmaceutical compositions can be used to treat, prevent or ameliorate respiratory condition, allergic condition, pain, inflammatory condition, mucus secretion disorder, bone disease, sleep disorder, fertility disease, blood coagulation disorder, vision problems, and immune and autoimmune diseases in mammals, especially humans. In addition, such compounds can also inhibit cell tumorigenic transformation and metastatic tumor growth, and thus can be used to treat various cancers. The pharmaceutically acceptable salt of the heterocyclic compound represented by formula A or the hydrate of the salt can also be used to treat and/or prevent prostaglandin-mediated proliferative diseases, such as, a proliferative disease that may occur in diabetic retinopathy and tumor angiogenesis. The pharmaceutically acceptable salt of the heterocyclic compound represented by formula A or the hydrate of the salt can also inhibit smooth muscle contraction induced by prostaglandin by antagonizing contractile prostanoids or simulating relaxant prostaglandins, and thus can be used to treat dysmenorrhea, premature birth and eosinophil-related disease. More specifically, the pharmaceutically acceptable salt of the heterocyclic compound represented by formula A or the hydrate of the salt is an antagonist of the prostaglandin D2 receptor (CRTH2).

The present invention also provides a method for antagonizing PGD₂ receptor including CRTH2 receptor, comprising administering an effective amount of the pharmaceutically acceptable salt of the heterocyclic compound represented by formula A or the hydrate of the salt to a mammalian in need.

In another aspect of the present invention, it provides a method of treating or preventing a prostaglandin-mediated disease, comprising administering the pharmaceutically acceptable salt of the heterocyclic compound represented by formula A or the hydrate of the salt in an amount effective to treat or prevent the prostaglandin-mediated disease to a mammalian patient in need of such treatment.

The compounds and compositions of the present invention can be used to treat prostaglandin-mediated diseases, including but not limited to allergic rhinitis, nasal congestion, runny nose, perennial rhinitis, rhinitis, asthma including allergic asthma, chronic obstructive pulmonary disease and other forms of pneumonia; sleep sickness and sleep-wake cycle disorder; dysmenorrhea and premature birth related to smooth muscle contraction induced by prostaglandin; eosinophil-related diseases; thrombosis; glaucoma and vision disorder; obliterative vascular disease; congestive heart failure; disease or condition that requires anticoagulant therapy, such as post-injury treatment or post-surgical treatment; inflammation; gangrene; Raynaud's disease; mucus secretion disorder including cell protection; pain and migraine; diseases that require control of bone formation and resorption, such as osteoporosis; shock; heat regulation including fever; and immune diseases or disorders that require immune regulation. More specifically, the disease to be treated is a disease mediated by prostaglandin D2, such as allergic rhinitis, pulmonary congestion, and asthma including allergic asthma.

The present invention also provides a method for treating or preventing a prostaglandin-mediated disease, comprising administering the pharmaceutically acceptable salt of the heterocyclic compound represented by formula A or the hydrate of the salt in an amount effective to treat or prevent the prostaglandin-mediated disease to a mammalian patient in need of such treatment, wherein the prostaglandin-mediated disease is nasal congestion, rhinitis including allergic rhinitis and perennial rhinitis, and asthma including allergic asthma.

The present invention also provides a method for treating or preventing a prostaglandin D2-mediated disease, comprising administering the pharmaceutically acceptable salt of the heterocyclic compound represented by formula A or the hydrate of the salt in an amount effective to treat or prevent the prostaglandin D2-mediated disease to a mammalian patient in need of such treatment, wherein the prostaglandin D2-mediated disease is nasal congestion or asthma.

The present invention also provides a method for treating nasal congestion in a patient in need of such treatment, comprising administering a therapeutically effective amount of the pharmaceutically acceptable salt of the heterocyclic compound represented by formula A or the hydrate of the salt to the patient.

The present invention also provides a method for treating asthma, especially allergic asthma, in a patient in need of such treatment, comprising administering a therapeutically effective amount of the pharmaceutically acceptable salt of the heterocyclic compound represented by formula A or the hydrate of the salt to the patient.

Those skilled in the art will readily understand that the compounds disclosed herein can be mixed with pharmaceutically acceptable excipients well known in the art for administration. Specifically, as a systemic drug, it can be formulated into a capsule, powder, pill, tablet or the like suitable for oral or parenteral administration or inhalation.

In order to treat and prevent a disease mediated by prostaglandin, the pharmaceutically acceptable salt of the heterocyclic compound represented by formula A or the hydrate of the salt may be co-administered with other therapeutic agents. Therefore, another aspect of the present invention provides a pharmaceutical composition for the treatment of prostaglandin-mediated disease, which comprises a therapeutically effective amount of the pharmaceutically acceptable salt of the heterocyclic compound represented by formula A or the hydrated salt of the salt, and one or more other therapeutic agents. Suitable therapeutic agents used in combination therapy with the pharmaceutically acceptable salt of the heterocyclic compound represented by formula A or the hydrate of the salt include: (1) DP receptor antagonist, such as S-5751 or Laropiprant; (2) corticosteroid, such as triamcinolone acetonide; (3) β-agonist, such as salmeterol, formoterol, terbutaline, metaproterenol, albuterol, etc.; (4) leukotriene modifier, including leukotriene receptor antagonist or lipooxygenase inhibitor, such as montelukast, zafirlukast, pranlukast or zileuton; (5) antihistamine, such as bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline , pheniramine, pyrilamine, astemizole, terfenadine, loratadine, cetirizine, fexofenadine and descarboethoxyloratadine, etc.; (6) decongestant, including phenylephrine, phenylpropanolamine, pseudophedrine, oxymetazoline, ephinephrine, naphazoline, xylometazoline, propylhexedrine or levo-desoxyephedrine; (7) antiitussive, including codeine, hydrocodone, caramiphen, carbetapentane or dextramethorphan; (8) another prostaglandin ligand, including prostaglandin F agonist, such as latanoprost, misoprostol, enprostil, rioprostil, omoprostol or rosaprostol; (9) diuretic; (10) non-steroidal anti-inflammatory agent (NSAID), such as propionic acid derivatives (alminoprofen, benoxaprofen, bucloxicacid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin and zomepirac), fenamic acid derivatives (flufenamic acid, meclofenamic acid, mefenamic acid, niflumic acid and tofenamic acid), biphenylcarboxylic acid derivatives (diflunisal and flufenisal), oxicams (isoxicam, piroxicam, sudoxicam and tenoxican), salicylic acids (acetylsalicylic acid, sulfasalazine) and pyrazolone (apazone, bezpiperylon, feprazone, mofebutazone, oxyphenbutazone, phenylbutazone); (11) cyclooxygenase-2 (COX-2) inhibitors, such as celecoxib and rofecoxib; (12) phosphodiesterase IV (PDE-IV) inhibitors, such as Ariflo, roflumilast; (13) antagonists of chemokine receptors, especially CCR-1, CCR-2 and CCR-3; (14) cholesterol-lowering agents, such as HMG-CoA reductase inhibitors (lovastatin, simvastatin and pravastatin, fluvastatin, atorvastatin and other statins), chelating agents (cholestyramine and colestipol), niacin, fenofibric acid derivatives (gemfbrozil, clofibrat, fenofibrate, bezafibrate, and probucol; (15) antidiabetic drugs, such as insulin, sulfonylureas, biguanides (metformin), α-glucosidase inhibitors (acarbose) and glitazones (troglitazone, pioglitazone, englitazone and rosiglitazone, etc.); (16) preparations of interferon β (interferon β-1a, interferon β-1b); (17) anticholinergics, such as muscarinic antagonists (ipratropium bromide and tiotropium bromide), and selective muscarinic M3 antagonists; (18) steroids, such as beclomethasone, methylprednisolone, betamethasone, prednisone, dexamethasone, and hydrocortisone; (19) triptans commonly used in the treatment of migraine, such as sumitriptan and rizatriptan; (20) alendronate and other osteoporosis therapeutics; (21) other compounds, such as 5-aminosalicylic acid and prodrug thereof, antimetabolites, such as azathioprine and 6-mercaptopurine, cytotoxic cancer chemotherapeutics, bradykinin (BK2) antagonists, such as FK-3657, TP receptor antagonists, such as seratrodast, neurokinin antagonists (NK1/NK2), VLA-4 antagonists, for example those antagonists described in US 5,510,332, WO97/03094, WO97/02289, WO96/40781, WO96/22966, WO96/20216, WO96/01644, WO96/06108, WO95/15973 and WO96/31206.

The present invention also provides a use of at least one of the heterocyclic compound represented by formula A, or the pharmaceutically acceptable salt or hydrate thereof as described above, in the preparation of a medicament for the treatment of acute lung injury or acute respiratory distress syndrome,

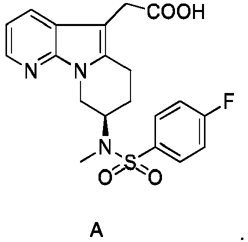

According to an embodiment of the present invention, the acute lung injury is selected from cigarette smoke (CS) or lipopolysaccharide (LPS) induced acute lung injury.

According to the technical solution of the present invention, the pharmaceutically acceptable salt of the heterocyclic compound represented by formula A is selected from alkali metal salts.

According to the technical solution of the present invention, the hydrate is selected from the hydrate of the heterocyclic compound represented by formula A, or the hydrate of the alkali metal salt of the heterocyclic compound represented by formula A.

According to a preferred technical solution of the present invention, the heterocyclic compound represented by formula A or the pharmaceutically acceptable salt thereof is in a crystalline form, such as the crystal form of the heterocyclic compound represented by formula A, the crystal form of hydrate thereof, the crystal form of the pharmaceutically acceptable alkali metal salt thereof or the crystal form of the hydrate of the alkali metal salt.

According to an embodiment of the present invention, the crystalline form of the heterocyclic compound represented by formula A or the hydrate thereof has characteristic peaks at 2θ angles of 11.1±0.2°, 11.4±0.2°, 17.9±0.2, 22.6±0.2°, and 24.4±0.2°in the X-ray powder diffraction spectrum using Cu-Ka radiation.

Preferably, the crystal form has characteristic peaks at 2θ angles of 8.6±0.2°, 11.1±0.2°, 11.4±0.2°, 14.1±0.2°, 16.1±0.2°, 17.9±0.2°, 20.9±0.2°, 22.6±0.2°, 24.4±0.2°, and 25.8±0.2°in the X-ray powder diffraction spectrum using Cu-Ka radiation.

More preferably, the crystal form has characteristic peaks at 2θ angles of 8.6±0.2°, 11.1±0.2°, 11.4±0.2°, 14.1±0.2°, 15.6±0.2°, 16.1±0.2°, 17.9±0.2°, 18.3±0.2°, 20.9±0.2°, 22.6±0.2°, 24.4±0.2°, 25.8±0.2°, 26.5±0.2°, and 28.9±0.2°in the X-ray powder diffraction spectrum using Cu-Ka radiation.

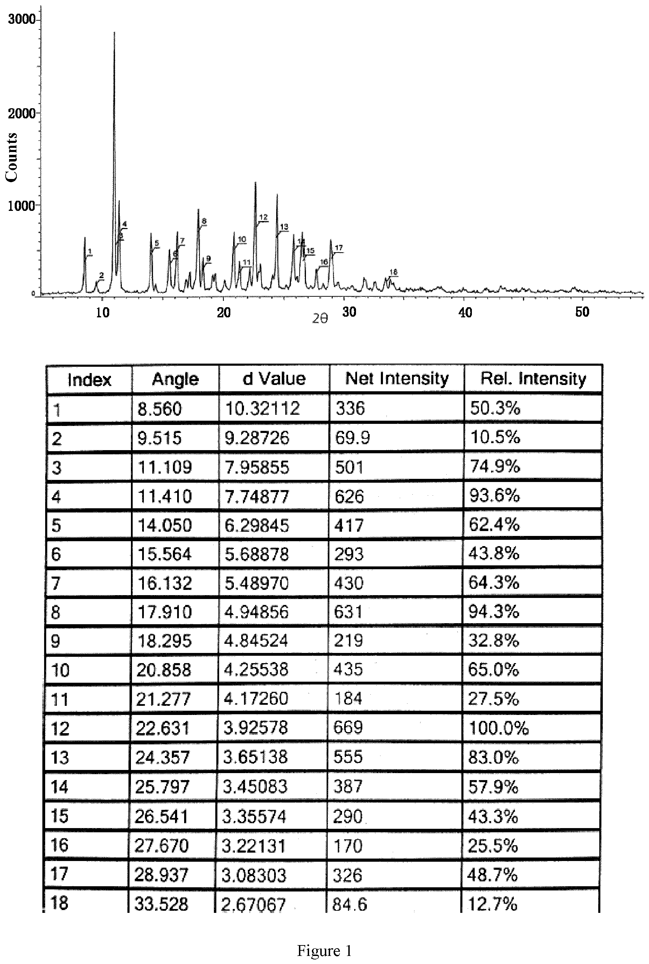

Further preferably, the crystal form has an X-ray powder diffraction spectrum substantially as shown in FIG. 1.

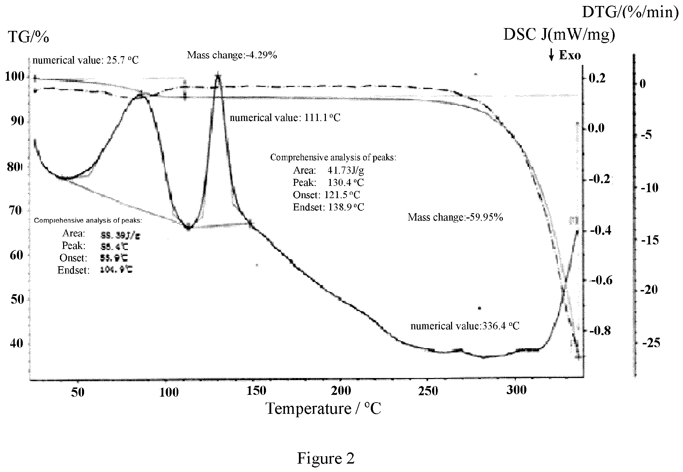

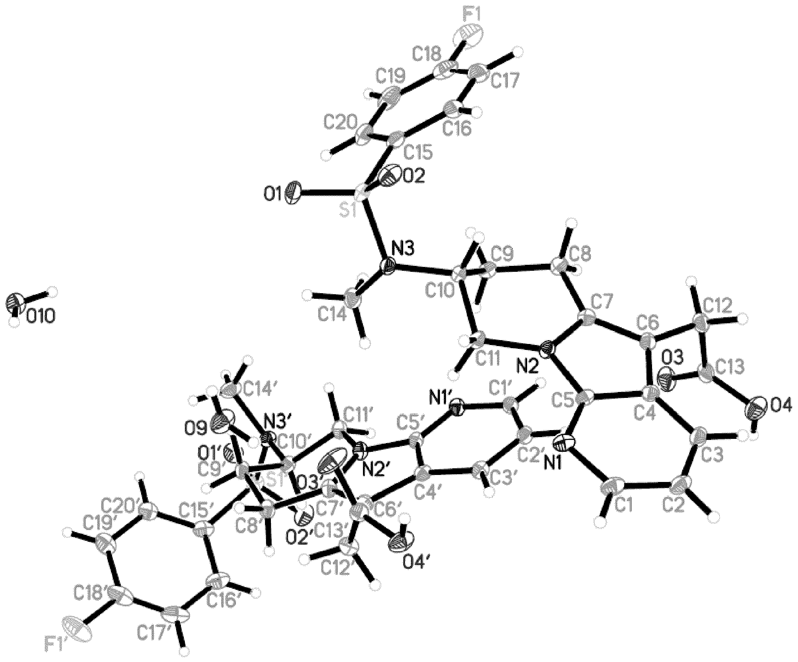

More preferably, the crystal form has a DSC-TGA spectrum substantially as shown in FIG. 2.

According to the present invention, the crystal form of the hydrate of the heterocyclic compound represented by formula A is preferably a monohydrate. More preferably, the mass fraction of water in the hydrate is 4.2-5.2%, more preferably 4.5-5.0%.

The monohydrate is as follows:

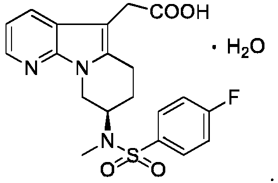

Still more preferably, the crystal form is a single crystal having the following single crystal parameters:

| | |
|---|---|
| C₂₀ H₂₂ F N₃ O₅ S | *V*=3937.1(13)Å³ |
| *Mr*=435.47 | Z=8 |
| monoclinic crystal system, C2 | *Dₓ*=1.469mg/m³ |
| *a* = 17.022(3)Å | Mo Kα radiation (λ = 0.71073_) |
| *b* = 11.380(2)A | *θ*=2.20°-28.0° |
| c = 21.738(4)Å | *µ*=0.213mm⁻¹ |
| *α*=90° | *T*=153(2)k |
| *β*=110.783(2))° | Bulk, colorless |
| *γ*=90° | 0.50×0.34×0.21mm |

The present invention also provides a method for preparing the crystal form of the heterocyclic compound represented by formula A or the hydrate thereof comprising the following steps:
placing the compound represented by formula A in a mixed solvent composed of a ketone solvent and water; heating to dissolve and then cooling, stirring and crystallizing to obtain the crystal form of the heterocyclic compound represented by formula A or the hydrate thereof,

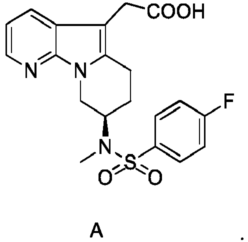

The heterocyclic compound represented by formula A in the present invention can be prepared by referring to the methods described in Examples 3 and 4 in CN101896178B. For example, the racemate of the heterocyclic compound represented by formula A is eluted with a methanol solution containing 0.05% trifluoroacetic acid on a Chiralcel OJ-RH column (Chiralcel Technologies) to separate the heterocyclic compound represented by formula A.

According to the preparation method of the present invention, the ketone solvent is selected from acetone or methyl ethyl ketone.

According to the preparation method of the present invention, the volume ratio of the ketone solvent to water is (1-3):1, for example, 1:1.

According to the preparation method of the present invention, the heating temperature is 30 to 80°C, and preferably 40 to 60°C.

According to an embodiment of the present invention, the pharmaceutically acceptable alkali metal salt of the heterocyclic compound represented by formula A is preferably sodium salt, a lithium salt or a potassium salt; and the hydrate of the alkali metal salt is selected from the hydrate of sodium salt, lithium salt or potassium salt.

Preferably, the crystal form of the alkali metal salt of the compound of formula A is in the form of a hydrate.

According to the present invention, the crystalline hydrate of the alkali metal salt of the compound of formula A is preferably a monohydrate.

As an example, the crystalline hydrate of the alkali metal salt of the compound of formula A is selected from a compound represented by the following formulas A-N, A-L or A-K:

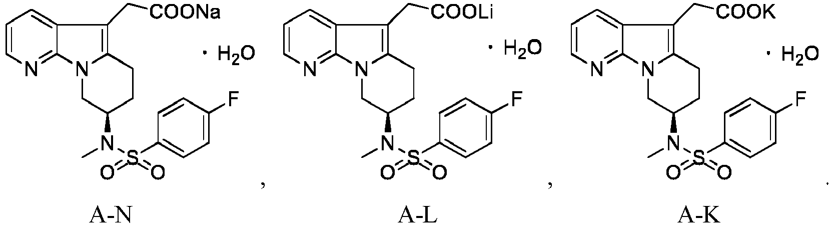

Preferably, the compound represented by formula A-N is a crystalline hydrate, which has characteristic peaks at 2θ angles of 16.4±0.2°, 18.9±0.2°, 21.7±0.2°, and 24.0±0.2° in the X-ray powder diffraction spectrum using Cu-Ka radiation.

Preferably, it has characteristic peaks at 2θ angles of 11.8±0.2°, 16.4±0.2°, 16.7±0.2°, 16.9±0.2°, 17.1±0.2°, 17.8±0.2°, 18.6±0.2°, 18.9±0.2°, 21.7±0.2°, 23.7±0.2°, and 24.0±0.2° in the X-ray powder diffraction spectrum using Cu-Ka radiation.

More preferably, the crystalline hydrate has characteristic peaks at 2θ angles of 5.6±0.2°, 11.8±0.2°, 14.0±0.2°, 15.8±0.2°, 16.4±0.2°, 16.7±0.2°, 16.9±0.2°, 17.1±0.2°, 17.8±0.2°, 18.6±0.2°, 18.9±0.2°, 20.3±0.2°, 21.7±0.2°, 23.7±0.2°, 24.0±0.2°, 26.1±0.2°, 28.1±0.2°, 28.5±0.2°, and 29.8±0.2° in the X-ray powder diffraction spectrum using Cu-Ka radiation.

Further preferably, the crystalline hydrate has an X-ray powder diffraction spectrum (XRPD) substantially as shown in FIG. 4.

Preferably, the mass fraction of water in the crystal hydrate of the compound represented by formula A-N is 3.4-4.4%, and more preferably 3.6-4.2%.

More preferably, the crystalline hydrate of the compound represented by formula A-N has a DSC-TGA spectrum substantially as shown in FIG. 5.

According to the present invention, the potassium salt compound represented by formula A-K is a crystalline hydrate, which has characteristic peaks at 2θ angles of 15.6±0.2°, 21.4±0.2°, and 24.0±0.2° in the X-ray powder diffraction spectrum using Cu-Ka radiation.

Preferably, the potassium salt compound represented by formula A-K is a crystalline hydrate, which has characteristic peaks at 2θ angles of 11.7±0.2°, 15.6±0.2°, 16.6±0.2°, 17.9±0.2°, 18.5±0.2°, 21.4±0.2°, 24.0±0.2°, and 28.2±0.2° in the X-ray powder diffraction spectrum using Cu-Ka radiation.

Preferably, the potassium salt compound represented by formula A-K is a crystalline hydrate, which has characteristic peaks at 2θ angles of 11.7±0.2°, 15.6±0.2°, 15.9±0.2°, 16.6±0.2°, 17.4±0.2°, 17.9±0.2°, 18.5±0.2°, 21.4±0.2°, 23.5±0.2°, 24.0±0.2°, 27.7±0.2°, and 28.2±0.2° in the X-ray powder diffraction spectrum using Cu-Ka radiation.

More preferably, the crystalline hydrate of the compound represented by formula A-K has characteristic peaks at 2θ angles of 11.7±0.2°, 14.0±0.2°, 15.6±0.2°, 15.9±0.2°, 16.6±0.2°, 17.4±0.2°, 17.9±0.2°, 18.5±0.2°, 20.1±0.2°, 21.4±0.2°, 23.5±0.2°, 24.0±0.2°, 27.5±0.2°, 27.7±0.2°, 28.2±0.2°, 28.6±0.2°, 29.3±0.2°, and 29.6±0.2° in the X-ray powder diffraction spectrum using Cu-Ka radiation.

Further preferably, the crystalline hydrate of the compound represented by formula A-K has an X-ray powder diffraction spectrum substantially as shown in FIG. 6.

Preferably, the mass fraction of water in the crystal hydrate of the compound represented by formula A-K is 3.3-4.3%, and more preferably 3.5-4.1%.

Further preferably, the crystalline hydrate of the compound represented by formula A-K has a DSC-TGA spectrum substantially as shown in FIG. 7.

According to the present invention, the lithium salt compound represented by formula A-L is a crystalline hydrate, which has characteristic peaks at 2θ angles of 16.7±0.2°, 18.8±0.2°, 21.9±0.2°, and 23.9±0.2° in the X-ray powder diffraction spectrum using Cu-Ka radiation.

More preferably, the crystalline hydrate of the compound represented by formula A-L has characteristic peaks at 2θ angles of 5.6±0.2°, 8.8±0.2°, 11.8±0.2°, 14.0±0.2°, 16.3±0.2°, 16.7±0.2°, 16.9±0.2°, 17.0±0.2°, 17.7±0.2°, 18.5±0.2°, 18.8±0.2°, 21.9±0.2°, 23.9±0.2°, and 28.2±0.2° in the X-ray powder diffraction spectrum using Cu-Ka radiation.

Further preferably, the crystalline hydrate of the compound represented by formula A-L has an X-ray powder diffraction spectrum substantially as shown in FIG. 8.

Preferably, the mass fraction of water in the crystal hydrate of the compound represented by formula A-L is 3.6-4.6%, and more preferably 3.9-4.5%.

More preferably, the crystalline hydrate of the compound represented by formula A-L has a DSC-TGA spectrum substantially as shown in FIG. 9.

The present invention also provides a method for preparing the pharmaceutically acceptable salt of the heterocyclic compound represented by formula A or the hydrate of the salt, comprising the following steps:
dissolving the compound represented by formula A in a ketone solvent; adding an aqueous solution of alkali metal hydroxide to the ketone solvent for reaction; then filtering and drying to obtain it; wherein the alkali metal hydroxide is preferably sodium hydroxide, lithium hydroxide or potassium hydroxide. The heterocyclic compound represented by formula A in the present invention can be prepared by referring to the methods described in Examples 3 and 4 in CN101896178B. For example, the racemate of the heterocyclic compound represented by formula A is eluted on a Chiralcel OJ-RH column (Chiralcel Technologies) with a methanol solution containing 0.05% trifluoroacetic acid to separate the heterocyclic compound represented by formula A.

Regarding the heterocyclic compound represented by formula A, pharmaceutically acceptable salt, hydrate of the present invention and preparation thereof, refer to Chinese application No.201910024238.0 filed on January 10, 2019, which is incorporated herein by reference in its entirety.

The present invention also provides a pharmaceutical composition for the treatment of acute lung injury or acute respiratory distress syndrome, which comprises a therapeutically effective amount of the heterocyclic compound represented by formula A of the present invention, the pharmaceutically acceptable salt or hydrate thereof as described above.

According to an embodiment of the present invention, the acute lung injury is selected from cigarette smoke (CS)-induced or lipopolysaccharide (LPS)-induced acute lung injury.

According to the technical solution of the present invention, the pharmaceutically acceptable salt of the heterocyclic compound represented by formula A is selected from alkali metal salts.

According to the technical solution of the present invention, the hydrate is selected from the hydrate of the heterocyclic compound represented by formula A, or the hydrate of the alkali metal salt of the heterocyclic compound represented by formula A.

According to a preferred technical solution of the present invention, the heterocyclic compound represented by formula A or the pharmaceutically acceptable salt thereof is in a crystalline form, for example is selected from at least one of the crystal form of the heterocyclic compound represented by formula A, the crystal form of the hydrate thereof, the crystal form of the pharmaceutically acceptable alkali metal salt thereof or the crystal form of the hydrate of the alkali metal salt.

According to an embodiment of the present invention, the pharmaceutical composition further comprises at least one pharmaceutically acceptable carrier. The carrier may be an inert, non-toxic excipient, vehicle or diluent, for example, the carrier is selected from one, two or more of the following group consisting of disintegrant, glidant, lubricant, filler, adhesive, coloring agent, effervescent agent, flavoring agent, preservative, and coating material.

The present invention also provides a method for the treatment of acute lung injury or acute respiratory distress syndrome, comprising administering an therapeutically effective amount of the heterocyclic compound represented by formula A, the above-mentioned pharmacologically acceptable salt or hydrate thereof to a mammalian patient in need of such treatment.

The present invention also provides a method for treating acute lung injury or acute respiratory distress syndrome, comprising administering an therapeutically effective amount of the above-mentioned pharmaceutical composition to a mammalian patient in need of such treatment.

According to an embodiment of the present invention, the acute lung injury is selected from CS-induced or LPS-induced acute lung injury.

According to the technical solution of the present invention, the pharmaceutically acceptable salt of the heterocyclic compound represented by formula A is selected from alkali metal salts.

According to the technical solution of the present invention, the hydrate is selected from the hydrate of the heterocyclic compound represented by formula A, or the hydrate of the alkali metal salt of the heterocyclic compound represented by formula A.

According to a preferred technical solution of the present invention, the heterocyclic compound represented by formula A or the pharmaceutically acceptable salt thereof is in a crystalline form, for example selected from at least one of the crystal form of the heterocyclic compound represented by formula A, the crystal form of the hydrate thereof, the crystal form of the pharmaceutically acceptable alkali metal salt thereof or the crystal form of the hydrate of the alkali metal salt.

### Beneficial effect

The present invention first provides the sodium salt, potassium salt or lithium salt of the compound of formula A, and the hydrate thereof. The inventors have unexpectedly discovered that the solubility and dissolution of the three salts are improved compared to the compound of formula A, especially the solubility and dissolution of the sodium salt, lithium salt and the hydrate of both salts are significantly improved compared to the compound of formula A. Therefore, they are easier to prepare a medicine than the compound of formula A. In addition, sodium salt, lithium salt and the hydrate of both salts also have significantly better stability than the compound of formula A.

The inventors have also found that the hydrates of the sodium, lithium, and potassium salts of the compound of formula A can be prepared into crystalline forms, thereby indicating that the sodium, lithium, and potassium salts of the compound of formula A or the hydrate of the salt are more suitable for the development of medicine compared to the compound of formula A.

The present invention also provides the use of the heterocyclic compound represented by formula A, the hydrate, the pharmaceutically acceptable alkali metal salt thereof, or the hydrate of the alkali metal salt in the preparation of a medicament for the treatment of acute lung injury or acute respiratory distress syndrome. Experimental results show that the above compounds inhibit the penetration of macrophages and neutrophils into the lungs, reduce pulmonary vascular permeability, reduce the production of pro-inflammatory cytokines and cytokine chemokines, and promote IL-10 production, thereby greatly alleviating acute lung injury or acute respiratory distress syndrome induced by cigarette smoke (CS) or LPS. Moreover, the compounds can stop or alleviate inflammatory lung injury, reduce pulmonary edema, and ensure tissue oxygen supply, which show that they have a better treatment or relief effect for acute respiratory distress syndrome.

### Description of the drawings

Figure 1 is an XRPD spectrum of the crystal form obtained in Preparation Example 2.
Figure 2 is a DSC-TGA spectrum of the crystal form obtained in Preparation Example 2.

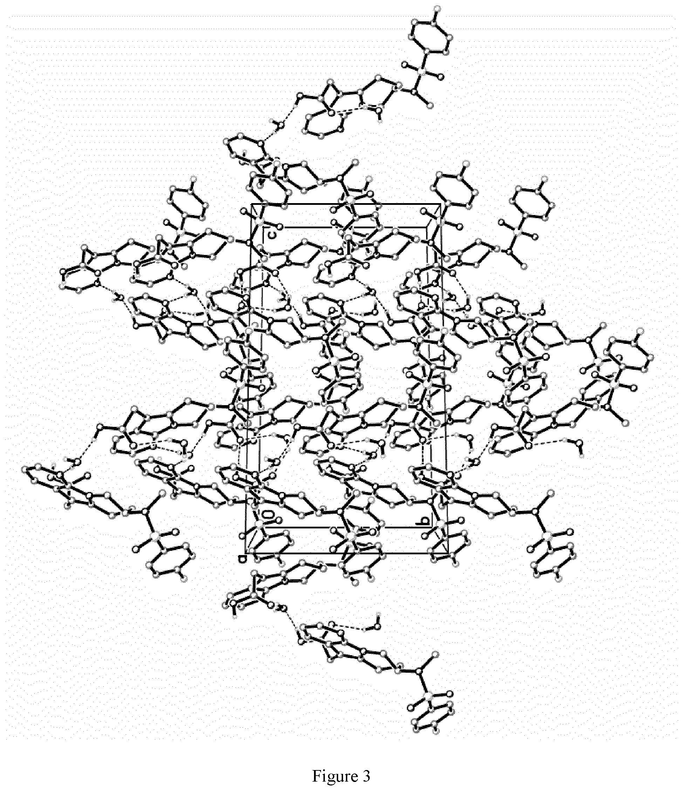

Figure 3 is a three-dimensional structure diagram and a unit cell diagram of the crystal form obtained in Preparation Example 2.
Figure 4 is an XRPD spectrum of the hydrate of the sodium salt obtained in Preparation Example 3.
Figure 5 is a DSC-TGA spectrum of the hydrate of the sodium salt obtained in Preparation Example 3.
Figure 6 is an XRPD spectrum of the hydrate of the potassium salt obtained in Preparation Example 4.
Figure 7 is a DSC-TGA spectrum of the hydrate of the potassium salt obtained in Preparation Example 4.
Figure 8 is an XRPD spectrum of the hydrate of the lithium salt obtained in Preparation Example 5.
Figure 9 is a DSC-TGA spectrum of the hydrate of the lithium salt obtained in Preparation Example 5.

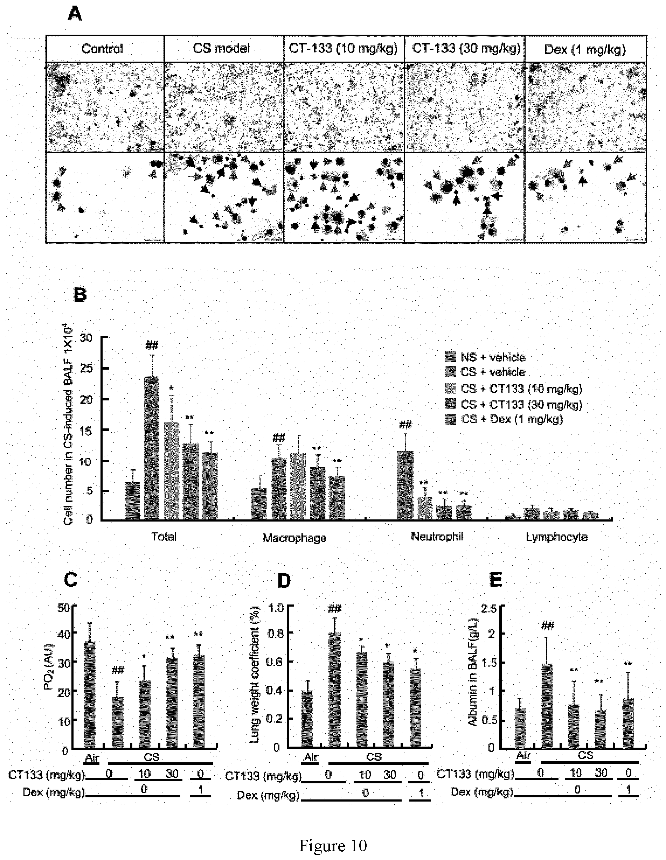

Figure 10 shows the effect of CT-NA on the number of inflammatory cells in BALF, partial pressure of oxygen (PO₂), lung weight coefficient and albumin content in BALF. Mice were intragastrically administered CT-NA (10 and 30 mg/kg), normal saline, or Dex (1 mg/kg) 1 hour before being exposed to CS for seven consecutive days. BALF was collected 24 hrs after the last CS exposure. (A) The images of neutrophils (black arrows) and macrophages (grey arrows) in the collected BALF. (B) Infiltration characteristics of total cells, macrophages, neutrophils and lymphocytes in BALF (from left to right are the vehicle saline group, vehicle CS exposure group, CT-NA 10mg/kg CS exposure group, CT-NA 30mg/kg CS exposure group and dexamethasone (Dex) 1mg/kg CS exposure group). (C) 24 hrs after CS exposure, the partial pressure of oxygen (PO₂) of all mice was measured using a Moor VMS-OXY™ monitor (Moor Instruments, United Kingdom). (D) After aspirating the surface blood from the dissected lung tissues, the lung weight coefficient was measured by dividing the individual lung weight of each mouse by its total body weight. (E) The albumin concentration in BALF was measured using albumin assay kits. ##p<0.01, versus the control (Ctrl) group; ^{∗}p<0.05 and ^{∗∗}p<0.01, versus the model group (model). The values are expressed as mean ± SEM; n=12 (each group).

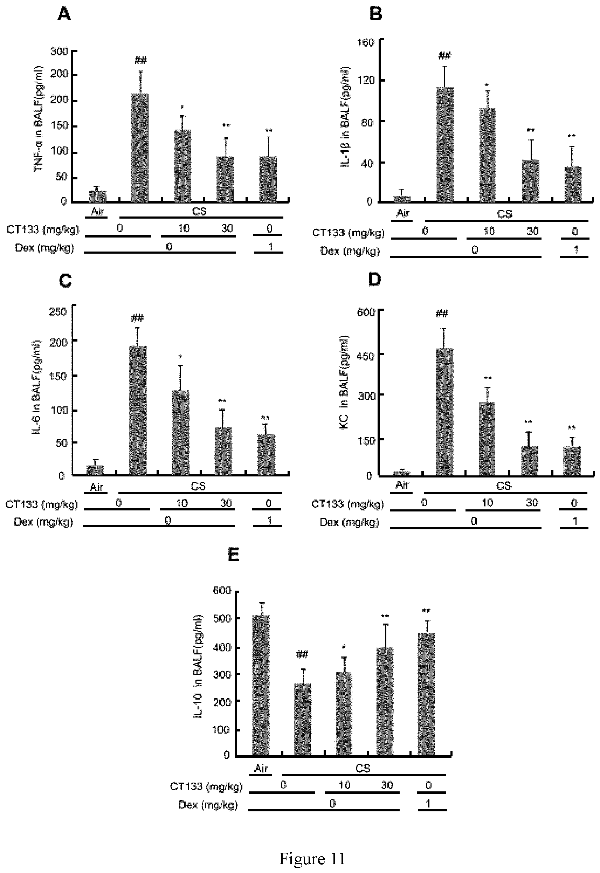

Figure 11 shows the effect of CT-NA on the expression of the pro-inflammatory cytokines (TNF-a, IL-1β, IL-6), chemokines (KC) and anti-inflammatory cytokine (IL-10) in BALF of CS-induced ALI mice. The collected BALF was tested by respective ELISA kits to analyze the expression levels of TNF-α (A), IL-1β (B), IL-6 (C), KC (D) and IL-10 (E). ##p<0.01, versus the control (Ctrl) group; ^{∗}p<0.05 and ^{∗∗}p<0.01, versus the model group (model). The values are expressed as mean ± SEM; n=12 (each group).

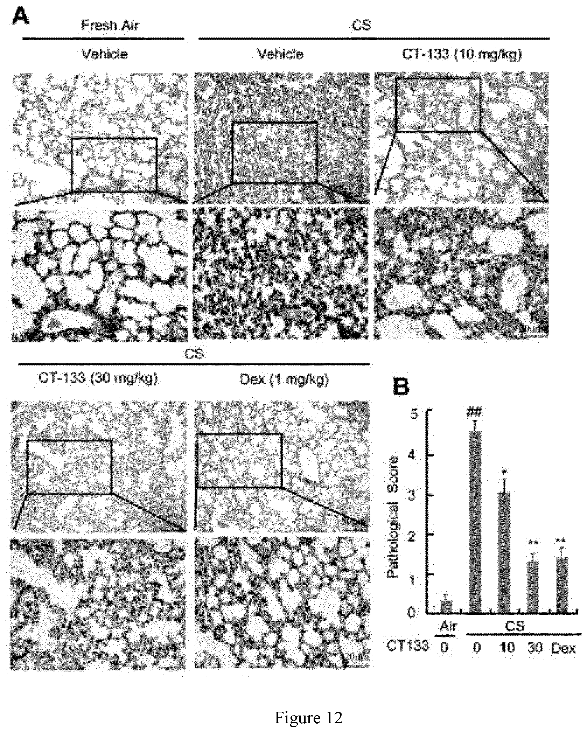

Figure 12 shows the effect of CT-NA on the histopathological changes in lung tissue of ALI mice induced by CS. The paraffin-embedded lung sections from each experimental group were stained with hematoxylin-eosin for histopathological evaluation. (A) Representative images of lung tissues stained with H&E to demonstrate the infiltration of macrophages, neutrophils and inflammatory cells. (B) Quantitative analysis of inflammatory cell infiltration and judgment of the severity of inflammation based on lung sections. ##p<0.01, versus the control (Ctrl) group; ^{∗}p<0.05 and ^{∗∗}p<0.01, versus the model group (model). The values are expressed as mean ± SEM; n=12 (each group).

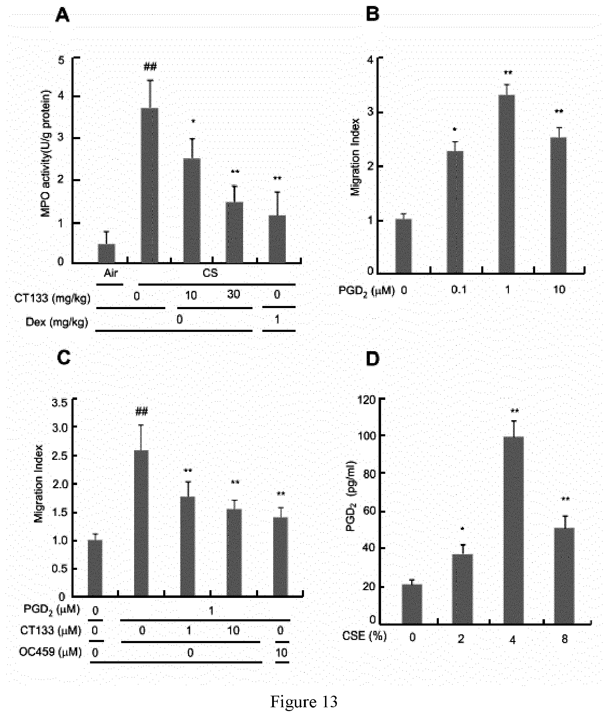

Figure 13 shows the effect of CT-NA on lung MPO activity and PGD₂-induced neutrophils migration *in vitro* and assessment of CSE-induced secretion of PGD₂ from primary macrophages. (A) The activity of MPO in lung homogenate was tested by MPO kits. The values are expressed as mean ± SEM; n=12 (each group). The PGD₂-induced neutrophil migration was detected in the absence (B) and presence (C) of CT-NA by Boyden chamber assay kits (3um pore size). (D) The isolated primary macrophages were treated with different concentrations of CSE (2%, 4%, and 8%) for 24 h, and then the supernatant was collected to evaluate the protein levels of PGD₂ extracellularly secreted by PGD₂ ELISA kits. ##p<0.01, versus the control (Ctrl) group; ^{∗}p<0.05 and ^{∗∗}p<0.01, versus the model group (model). All experiments were performed in triplicate wells under each condition and repeated three times. The values are expressed as mean ± SEM.

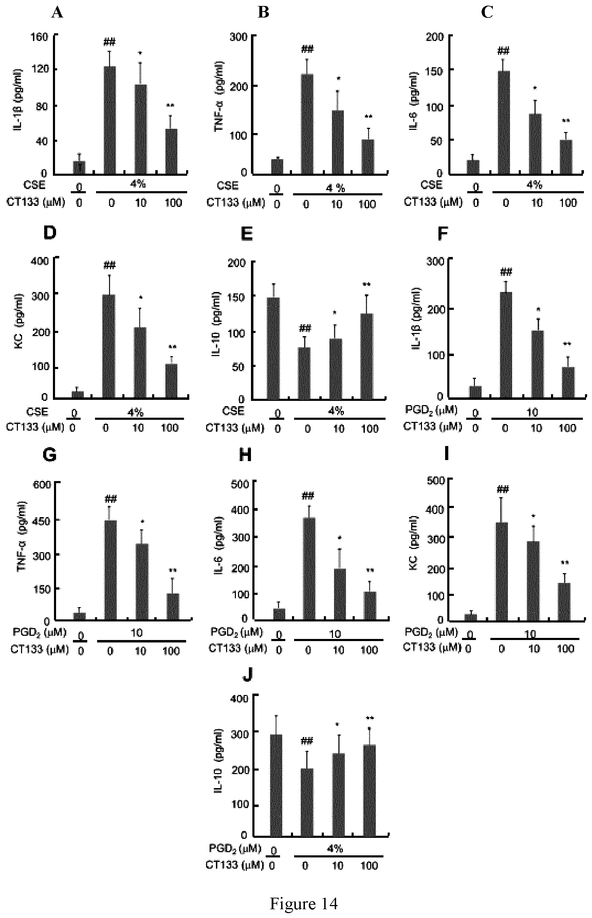

Figure 14 shows the effects of CT-NA on the protein levels of pro-inflammatory cytokines (TNF-a, IL-1β, IL-6) and chemokine (KC) and anti-inflammatory cytokine (IL-10) from RAW 264.7 macrophage induced by CSE (4%)and PGD₂. The supernatant was isolated from RAW 264.7 macrophages which were pretreated with CT-NA for 1 hour, and then treated with CSE/PGD₂ for 24 hrs, and then the protein levels of IL-1β (A and F), TNF-α (B and G), IL-6 (C and H), KC (D and I) and IL-10 (E and J) secreted extracellularly were measured using respective ELISA kits according to the instructions. ##p<0.01, versus the control (Ctrl) group; ^{∗}p<0.05 and ^{∗∗}p<0.01, versus PGD₂. All experiments were performed in triplicate wells under each condition and repeated three times. The values are expressed as mean ± SEM.

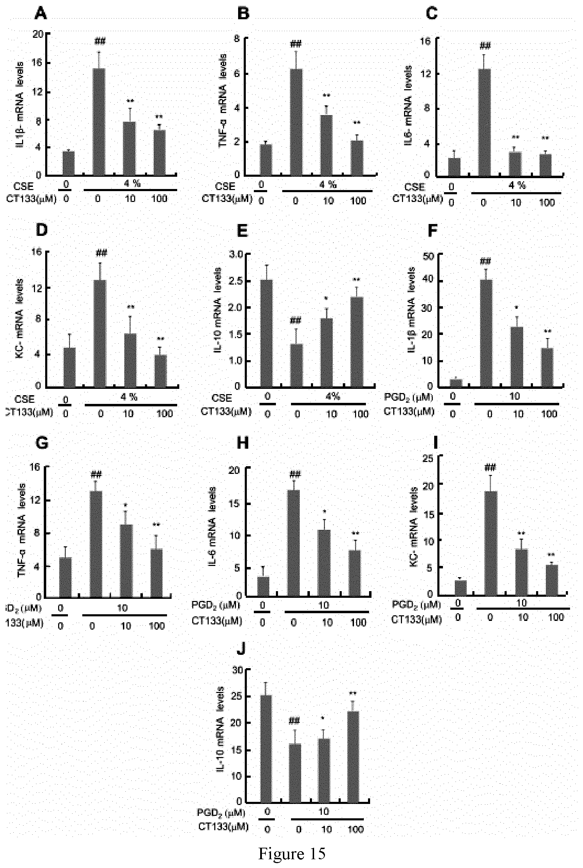

Figure 15 shows the effect of CT-NA on mRNA expressions of pro-inflammatory cytokines (TNF-a, IL-1β, IL-6), chemokine (KC) and anti-inflammatory cytokine (IL-10) from RAW 264.7 macrophage induced by CSE (4%) and PGD₂. RNAs was extracted from RAW 264.7 macrophages which were pretreated with CT-NA for 1 h, and then treated with CSE/PGD₂ for 24 h, and then the mRNA expression levels of IL-1β (A and F), TNF-α (B and G), IL-6 (C and H), KC (D and I) and IL-10 (E and J) were measured using RT-PCR respectively. ##p<0.01, versus the control (Ctrl) group; ^{∗}p<0.05 and ^{∗∗}p<0.01, versus PGD₂. All experiments were performed in triplicate wells for each condition and repeated three times. The values are expressed as mean ± SEM.

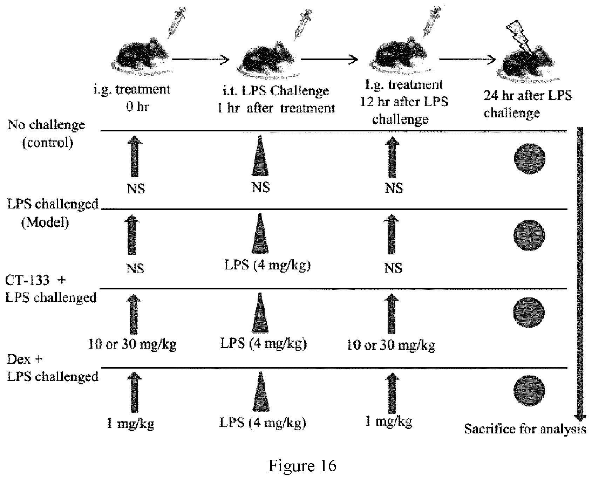

Figure 16 shows the process of preparing LPS-induced ALI mouse model. CT-NA (10 and 30 mg/kg) or Dex (positive control; 1 mg/kg) were intragastrically administered 1 hour before and 12 hrs after the intratracheal instillation of LPS. 24 hrs after LPS challenge, the mice were sacrificed to prepare BALF and lung tissue samples.

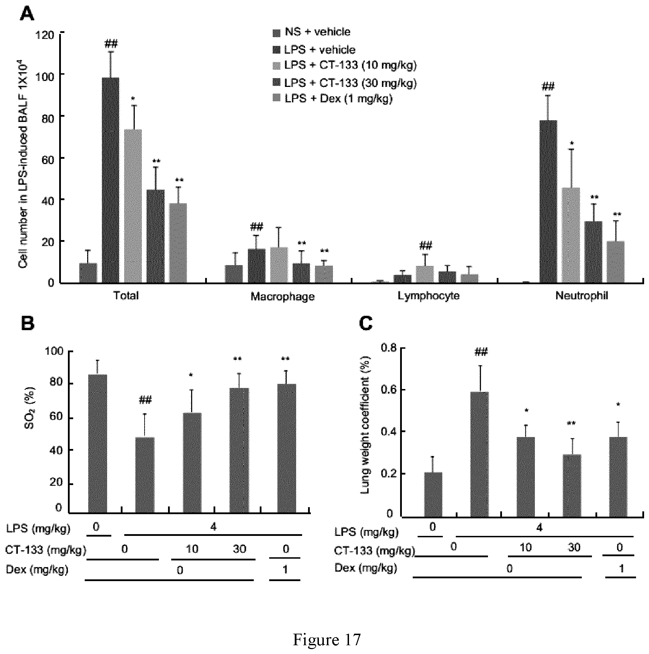

Figure 17 shows the effects of CT-NA on the count and classification of BALF inflammatory cells, oxygen saturation (SO₂) and lung weight coefficient. For acute lung injury, (A) CT-NA (10 and 30 mg/kg) or Dex (1 mg/kg) were intragastrically administered 1 hour before and 12 hrs after the intratracheal instillation of LPS. 24 hrs after LPS induction, BALF was collected to calculate the total number of cells, the number of neutrophils, macrophages and lymphocytes (from left to right in the total number of cells are the vehicle saline group, vehicle LPS induction group, LPS induction+CT-NA 10mg/kg group, LPS induction+CT-NA 30mg/kg group and LPS induction + dexamethasone (Dex) 1mg/kg group, the other test results are expressed in the same way as that for the total number of cells). (B) 24 hrs after LPS induction, the oxygen saturation (SO₂) was measured in all mice by using a Moor VMS-OXY™ monitor. (C) 24 hrs after LPS induction, the lung weight coefficient was measured by dividing the individual lung weight of each mouse by its body weight. ##P<0.01 versus NS + vehicle group; ^{∗}*P*<0.05, ^{∗∗}*P*<0.01 versus LPS + vehicle group The values are expressed as the mean ± S.E.M., n=12 for each group.

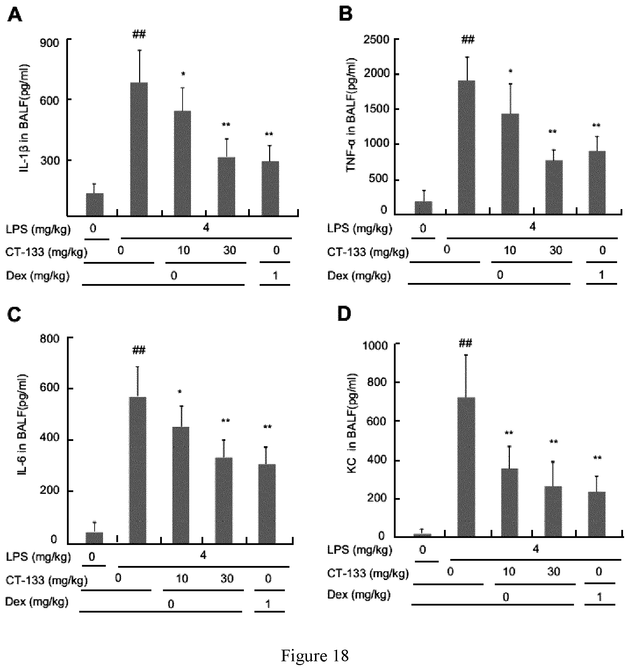

Figure 18 shows the effect of CT-NA on the production of pro-inflammatory cytokines (TNF-a, IL-1β, IL-6) and chemokines (KC) in BALF of LPS-induced ALI mice. BALF was collected and analyzed for the levels of TNF-α(A), IL-1β(B), IL-6(C) and KC(D) by using the corresponding ELISA kits. ##P<0.01 versus vehicle + vehicle group; ^{∗}*P*<0.05, ^{∗∗}*P*<0.01 versus LPS + vehicle group. The values are expressed as the mean ± S.E.M., n=12 for each group.

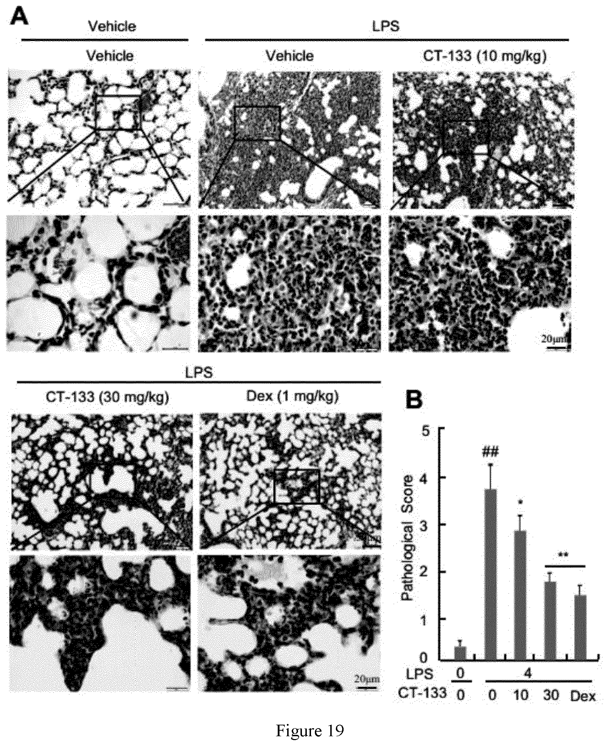

Figure 19 shows the effect of CT-NA on the histopathological changes of lung tissues in ALI mice induced by LPS. The paraffin-embedded lung sections of each experimental group were stained with H&E for histopathological analysis. (A) Representative images of lung tissues stained with H&E show edema, infiltration of neutrophil and inflammatory cells. (B) Quantitative analysis of lung injury scored by two blinded pathologists with expertise in lung pathology. ##P<0.01 versus vehicle + vehicle group; ^{∗}*P*<0.05, ^{∗∗}*P*<0.01 versus LPS + vehicle group. The values are expressed as mean ± S.E.M., n=12 for each group.

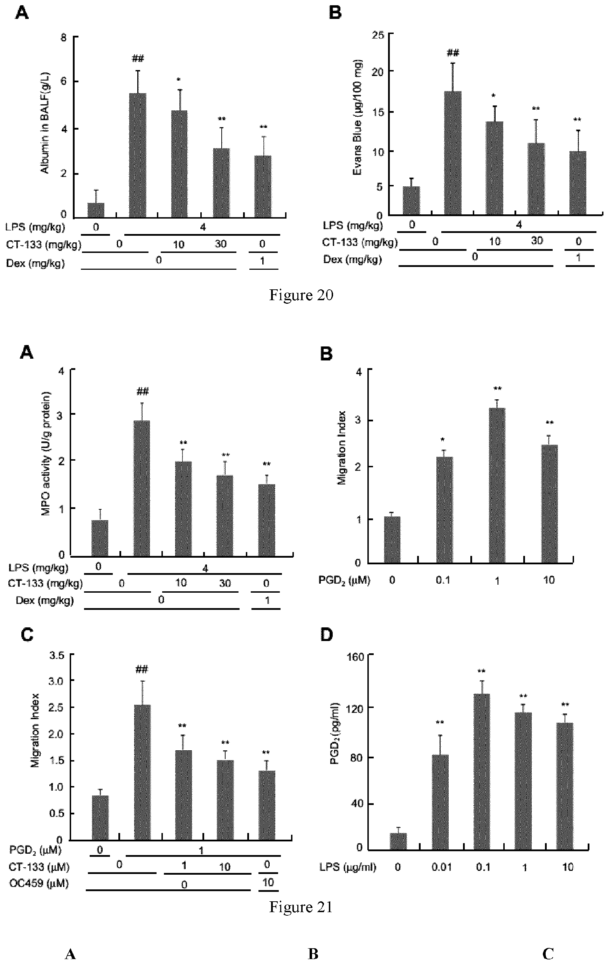

Figure 20 shows the effect of CT-NA on LPS-induced pulmonary vascular permeability. 24 hrs after LPS induction, (A) albumin in BALF was measured by using an albumin assay kit. (B) Evans blue dye (50 mg/kg) was injected into the caudal vein of all mice 1h before euthanasia. The pulmonary vascular permeability was determined by the accumulation of Evans blue dye in the lung tissue. ##P<0.01 versus vehicle + vehicle group; ^{∗}*P*<0.05, ^{∗∗}*P*<0.01 versus LPS + vehicle group. The values are expressed as mean ± S.E.M., n=12 for each group.
Figure 21 shows the effects of CT-NA on lung MPO activity and PGD₂-induced neutrophil migration *in vitro,* and evaluation of **LPS-induced secretion of PGD₂** from primary macrophages. (A) MPO activity of lung homogenate was measured using MPO kit. ##P<0.01 versus vehicle + vehicle group; ^{∗∗}*P*<0.01 versus LPS + vehicle group. The values are expressed as the mean ± S.E.M., n=12 for each group. (B and C) PGD₂-induced neutrophil migration was evaluated by Boyden chamber assay kit (3um pore size) in the presence or absence of CT-NA. ##P<0.01 versus vehicle + vehicle group; ^{∗}*P*<0.05, ^{∗∗}*P*<0.01 versus vehicle group or PGD2 +vehicle group. The values are expressed as the mean ± S.E.M., n=3 for each group. (D) The isolated primary macrophages were pretreated with different concentrations of LPS (0.01, 0.1, 1 and 10 µM) for 24 hrs, and then the supernatant was collected to measure the PGD₂ protein level with the PGD₂ ELISA kit. ##P<0.01 versus vehicle group. The values are expressed as the mean ± S.E.M., n=3 for each group.

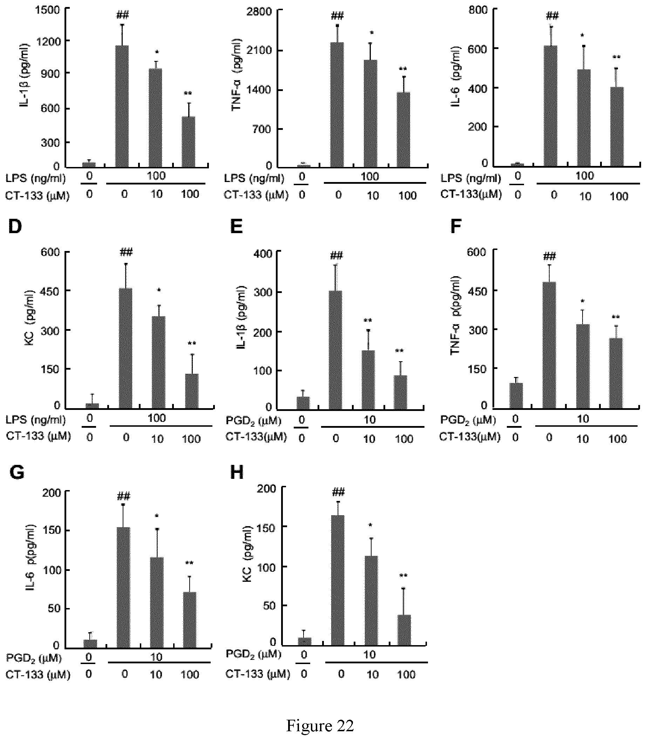

Figure 22 shows the effects of CT-NA on pro-inflammatory cytokines (TNF-a, IL-1β, IL-6) and chemokines (KC) secreted from LPS- or PGD₂ stimulated RAW264.7 macrophages. RAW264.7 macrophages were pretreated with CT-NA for 1 hour, and further treated with CT-NA and LPS/PGD₂ for 24 hrs, then the culture medium was collected to measure the levels of secreted IL-1β (A and E), TNF -α (B and F), IL-6 (C and G) and KC (D and H) using respective ELISA kits. ##P<0.01 versus vehicle + vehicle group; ^{∗}*P*<0.05, ^{∗∗}*P*<0.01 versus LPS/PGD2 +vehicle group. The values are expressed as the mean ± S.E.M., n=3 for each group.

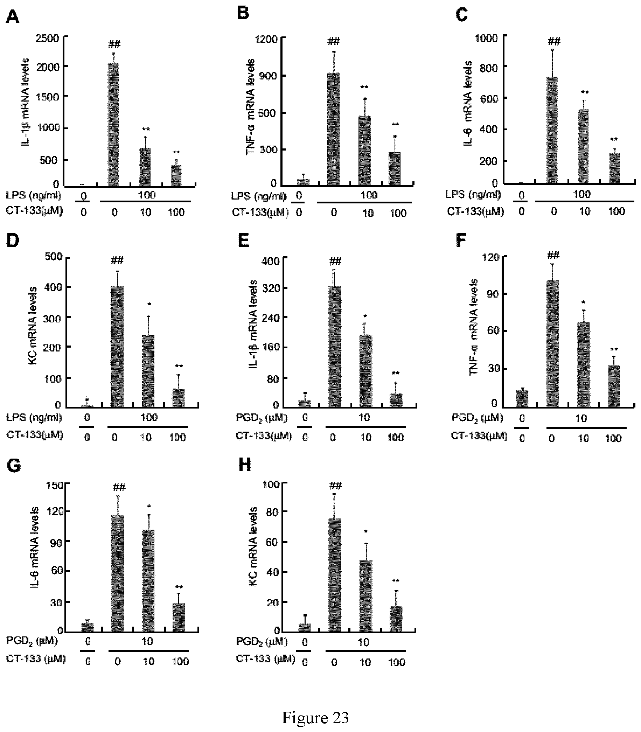

Figure 23 shows the effects of CT-NA on the mRNA expressions of chemokine (KC) and pro-inflammatory cytokines (TNF-a, IL-1β, IL-6) secreted by RAW264.7 macrophages stimulated by LPS- or PGD₂. RAW264.7 macrophages were pretreated with CT-NA for 1 h, and further treated with CT-NA and LPS/PGD₂ for 24 hrs, then RNA was extracted to measure the expressions of IL-1β (A and E), TNF-α (B and F), IL-6 (C and G) and KC (D and H) by RT-PCR. ##P < 0.01versus control group; ^{∗}P < 0.05, ^{∗∗}P < 0.01 versus model groups. ##P<0.01 versus vehicle +vehicle group; ^{∗}*P*<0.05, ^{∗∗}*P*<0.01 versus LPS/PGD2 + vehicle group. The values are expressed as the mean ± S.E.M., n=3 for each group.

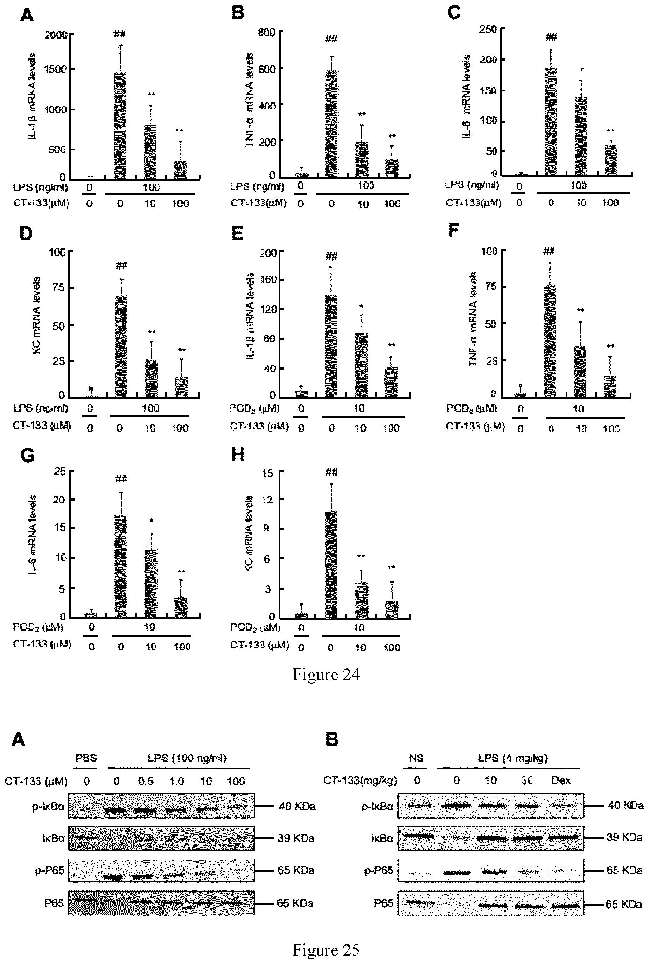

Figure 24 shows the effects of CT-NA on the mRNA expressions of pro-inflammatory cytokines (TNF-a, IL-1β, IL-6) and chemokine (KC) secreted by primary macrophages stimulated by LPS and PGD₂. The primary macrophages were pretreated with CT-NA for 1 hour, and further treated with CT-NA and LPS/PGD₂ for 24 hrs. Then total RNA was extracted for analysis of the expressions of IL-1β (A and E), TNF- α (B and F), IL-6 (C and G) and KC (D and H) by RT-PCR. ##P<0.01 versus vehicle + vehicle group; ^{∗}*P*<0.05, ^{∗∗}*P*<0.01 versus LPS/PGD2 + vehicle group. The values are expressed as the mean ± S.E.M., n=3 for each group.
Figure 25 shows the effects of CT-NA on the activation response of NF-κB signalling pathway in response to LPS stimulation in the lung or RAW 264.7 macrophages. (A) 1 hour before LPS (100ng/ml) treatment, RAW264.7 macrophages were pretreated with CT-NA (0.5, 1, 10 and 100 µM) for 1 h.(B) The preserved lung tissues were homogenized in RIPA buffer to extract total protein. Total proteins were subjected to western blot analysis with the indicated antibodies. β-actin was used as an internal control. All experiments were repeated at least three times.

The name and model of DSC-TGA test instrument: Synchronous thermal analyzer (STA449F3), 20°C to 350°C.

The name and model of XRPD test instrument in Preparation Example 2: AFC10/Saturn724+Rigaku X-ray diffraction analyzer.

The name and model of XRPD test instrument in Preparation Examples 3-5: D2 PHASER X-ray diffractometer (Bruck, Germany).

### Detailed description of the invention

The technical solution of the present invention will be further described in detail below in combination with specific examples. The following examples are only illustrative and explanation of the present invention, and should not be construed as limiting the scope of protection of the present invention. All technologies implemented based on the above contents of the present invention are within the scope of the present invention.

Unless otherwise specified, the raw materials and reagents used in the following examples are all commercially available products or can be prepared by known methods.

Preparation Example 1: Preparation of compound of formula A

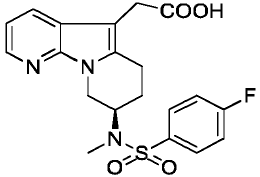

With reference to the methods described in Examples 3 and 4 in CN101896178B, the racemate of the heterocyclic compound represented by formula A (20 g) was eluted with methanol containing 0.05% TFA on a Chiralcel OJ-RH column (Chiralcel Technologies) to separate the heterocyclic compound represented by formula A (6.24 g, yield 31.2%).

¹H NMR(600MHz, CD₃OD): 8.14-8.15 (m, 1H), 8.00-8.03 (m, 2H), 7.92-7.94 (m, 1H), 7.38-7.42 (m, 2H),7.10-7.13 (m, 1H), 4.50-4.54 (m, 1H), 4.38-4.40 (m, 1H), 3.92-3.96 (m, 1H), 3.63-3.72 (m, 2H), 3.17-3.22 (m, 1H), 2.85-2.95 (m, 4H), 1.99-2.02 (m, 1H), 1.76-1.79 (m, 1H).

Preparation Example 2: Preparation of crystal form of monohydrate of the compound of formula A

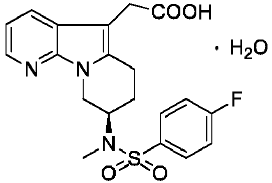

With reference to the methods described in Examples 3 and 4 in CN101896178B, the racemate of the heterocyclic compound of formula A (0.500g) was eluted with methanol containing 0.05% TFA on a Chiralcel OJ-RH column (Chiralcel Technologies), and the eluate was collected and concentrated to dryness to obtain about 0.2 g of the product, which was in an amorphous state. Acetone (2.5mL) and water (2.5mL) were added to the concentrate. After the mixture was heated at 40-50°C to dissolve and cooled to 0 - 10°C, it was stirred to crystallize for 2 - 3 hrs, and filtered to obtain the crystal form of the heterocyclic compound of formula A (0.156g) in a yield of 31.2%. The XRPD detection result of the crystal form is shown in Figure 1, and the DSC-TGA detection result is shown in Figure 2. It can be seen from the DSC spectrum of Figure 2 that it has endothermic peaks at 86.4°C and 130.4°C, respectively, and TGA thermal weight loss spectrum shows that the weight loss is 4.29%. The DSC-TGA pattern of the crystal form shows that the crystal form is a monohydrate. Figure 3 is a three-dimensional structure diagram and a unit cell diagram of the obtained crystal form.

¹H NMR (600MHz, CD₃OD): 8.13-8.15 (m, 1H), 8.00-8.04(m, 2H), 7.92-7.95(m, 1H), 7.37-7.42 (m, 2H), 7.09-7.14(m, 1H), 4.50-4.55(m, 1H), 4.38-4.42(m, 1H), 3.91-3.97(m, 1H), 3.61-3.71(m, 2H), 3.17-3.23(m, 1H), 2.85-2.95 (m, 4H), 1.97-2.02 (m, 1H), 1.75-1.79 (m, 1H).

Preparation Example 3: Preparation of hydrate of sodium salt of the compound of formula A (CT-NA)

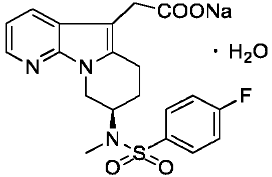

Compound A (17.6g) was dissolved in acetone (210mL), heated to 50°C and stirred to dissolve, and sodium hydroxide aqueous solution (prepared by dissolving 1.5g sodium hydroxide in 4mL purified water) was added dropwise into the solution. After addition, the mixture was stirred at 50°C for 4 hrs and then filtered. The filter cake was dried at 50 °C in vacuum under reduced pressure for 6 hrs to obtain the hydrate of sodium salt of compound A (15.8 g, yield 82%). The obtained sodium salt exhibits good crystallinity, and its XRPD characterization data is shown in Figure 4. The DSC-TGA detection result is shown in Figure 5. The DSC spectrum in Figure 5 shows that there are endothermic peaks at 130.2°C and 176.6°C, respectively, and the TGA thermal weight loss spectrum shows a weight loss of 3.99%. The DSC-TGA pattern of the crystal form shows that the crystal form is a monohydrate.

¹H NMR(600MHz, CD₃OD): 8.08-8.10 (m, 1H), 7.96-8.02 (m, 3H), 7.37-7.41 (m, 2H), 7.05-7.08 (m, 1H), 4.50-4.52 (m, 1H), 4.37-4.41 (m, 1H), 3.89-3.93 (m, 1H), 3.46-3.55(m, 2H), 3.17-3.22(m, 1H), 2.93(s, 3H), 2.90-2.92(m, 1H),1.96-2.00(m, 1H), 1.70-1.73(m, 1H).

Preparation Example 4: Preparation of hydrate of potassium salt of the compound of formula A

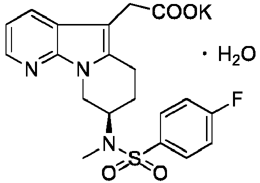

Compound A (50.0g) was dissolved in acetone (600mL), and potassium hydroxide aqueous solution (prepared by adding 6.4g potassium hydroxide into 12mL purified water) was slowly added dropwise into the reaction solution at room temperature. The reaction mixture was stirred at room temperature for 3h and then filtered under suction and dried under vacuum at 50°C for 6 hrs to obtain the hydrate of potassium salt of the compound A (36.4 g, yield 64%). The obtained potassium salt exhibits good crystallinity, and its XRPD spectrum is shown in Figure 6, and its DSC-TGA spectrum is shown in Figure 7. It can be seen from the DSC spectrum of Figure 7 that there are endothermic peaks at 64.1°C and 270.4°C, respectively, and the TGA thermal weight loss spectrum shows that the weight loss is 3.82%. The DSC-TGA pattern of the crystal form shows that the crystal form is a monohydrate.

¹H NMR(600MHz, DMSO-*d₆*): 7.98-8.05 (m, 3H), 7.80-7.83 (m, 1H), 7.48-7.52 (m, 2H), 6.94-6.97 (m, 1H), 4.30-4.33 (m, 1H), 4.16-4.20 (m, 1H), 3.81-3.86 (m, 1H), 3.06-3.17(m, 3H), 2.83(s, 3H), 2.76-2.82(m, 1H),1.82-1.90(m, 1H), 1.55-1.58(m, 1H).

Preparation Example 5: Preparation of hydrate of lithium salt of the compound of formula A

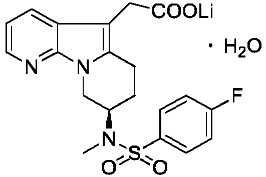

Compound A (50.0g) was dissolved in acetone (900mL), and an aqueous solution of lithium hydroxide (prepared by adding 2.75g of lithium hydroxide into 18mL of purified water) was slowly added dropwise into the reaction solution at room temperature. The reaction mixture was stirred at room temperature for 3 hrs and then filtered under suction. The filter cake was dried at 50° C in vacuum under reduced pressure for 6 hrs to obtain the hydrate of lithium salt of the compound A (26.0 g, yield 49%). The obtained lithium salt exhibits good crystallinity, and its XRPD spectrum is shown in Figure 8, and its DSC-TGA spectrum is shown in Figure 9. It can be seen from the DSC spectrum of Figure 9 that there are endothermic peaks at 138.8°C and 180.7°C, respectively, and the TGA thermal weight loss spectrum shows that the weight loss is 4.47%. The DSC-TGA pattern of the crystal form shows that the crystal form is a monohydrate.

¹H NMR(600MHz, DMSO-*d₆*): 7.98-8.06 (m, 3H), 7.81-7.83 (m, 1H), 7.47-7.52 (m, 2H), 6.91-6.94 (m, 1H), 4.28-4.34 (m, 1H), 4.15-4.19 (m, 1H), 3.80-3.84 (m, 1H), 3.18-3.26(m, 2H), 3.05-3.08(m, 1H), 2.82(s, 3H), 2.77-2.81(m, 1H),1.80-1.87(m, 1H), 1.53-1.56(m, 1H).

### Test example 1. Solubility test

The hydrates obtained as above and the compound of formula A were tested for solubility in solutions with different pH. The test method was as follows.
1.1. Preparation of the media with different pH
pH1.0 medium: 9.0mL of hydrochloric acid was diluted to 1000mL with water and shaken well to obtain the medium.
pH4.5 medium: 6.80g of potassium dihydrogen phosphate (KH₂PO₄) was dissolved in water and diluted to 1000mL, adjusted to pH4.5 with phosphoric acid or sodium hydroxide, and shaken well to obtain the medium.
pH6.8 medium: 55.38g of disodium hydrogen phosphate (Na₂HPO₄·12H₂O) and 4.77g of citric acid (C₆H₈O₇·H₂O) were dissolved in water and diluted to 1000mL, adjusted to pH6.8 with phosphoric acid or sodium hydroxide, and shaken well to obtain the medium.
Pure water medium: purified water.

1.2. Test method: To a certain amount of test samples, the media with different pH were added gradually, respectively. The mixture was shaken continuously until it reached saturation. The weighing amount of the test sample and the amount of solvent were recorded and the concentration when the sample was dissolved was calculated. The test results were shown in Table 1.

**Table 1. Solubility of compound A and salts thereof in water (unit: mg/mL)**

| Test results for solubility (mg/mL), at room temperature of 25°C | | | | |
|---|---|---|---|---|
| Sample name | pH=1.0 | pH=4.5 | pH=6.8 | purified water |
| Sodium salt hydrate | 0.067 | 0.005 | 0.222 | 12.5 |
| Potassium salt hydrate | 0.02 | 0.005 | 0.143 | 1.25 |
| Lithium salt hydrate | 0.033 | 0.005 | 0.2 | 12.5 |
| Free acid (Compound A) | 0.00125 | 0.00167 | 0.002 | 0.002 |

It can be seen from the results in Table 1 that sodium salt hydrate has the best water solubility at different pH. The solubility of lithium salt hydrate is basically equivalent to that of sodium salt hydrate. The solubility of potassium salt hydrate at pH 4.5 is equivalent to that of sodium salt hydrate and lithium salt hydrate, but its solubility in other pH and in purified water is worse than that of sodium salt hydrate and lithium salt hydrate. The free acid (compound A) has poor water solubility at different pH. The solubility of the obtained three salt hydrates, especially solubility of sodium salt hydrate and lithium salt hydrate is significantly better than that of compound A.

### Test example 2: Dissolution test

The hydrates obtained hereinabove and the compound of formula A were tested for dissolution rate. The test method was as follows.
Step 1: Pretreating raw materials
The samples were passed through a 100 mesh sieve.
Step 2: blending raw materials with auxiliary materials
The raw materials and lactose were weighed according to the prescription, mixed in equal increment, sieved and mixed.

| The capsule prescription of sodium salt hydrate | | The capsule prescription of potassium salt hydrate | | The capsule prescription of lithium salt hydrate | |
|---|---|---|---|---|---|
| Composition | 50 capsules/mg | Composition | 50 capsules/mg | Composition | 50 capsules/mg |
| Sodium salt monohydrate | 5.21 | Lithium salt monohydrate | 5.03 | Potassium salt monohydrate | 5.39 |
| Lactose F100 | 18.45 | Lactose F100 | 18.45 | Lactose F100 | 18.46 |

Step 3: Filling capsules
The capsules were filled via a capsule filling plate and sealed. The difference of the filling amount was controlled to ±5%. The appearance was inspected to ensure that the sealing was correct and there was no split or deformation.
Test method: dissolution test method (Chinese Pharmacopoeia, edited in 2015, Chapter 4, General Method 0931, the 2nd Method)
Dissolution medium: aqueous solution
Speed: 50rpm
Sampling time: 30 min

Specific test method: The sample was tested following the dissolution test method (Chinese Pharmacopoeia, edited in 2015, Chapter 4, General Method 0931, the 2nd Method). 900ml aqueous solution was used as the dissolution medium. The speed was 50rpm. The test was performed in accordance with the method. After 30min, about 10ml of the solution was taken out and filtered. After filtration, the filtrate was precisely measured and quantitatively diluted with the dissolution medium to prepare a solution containing about 10µg of sample per 1ml. The absorbance was measured by at 231nm by UV-Vis spectrophotometry (Chinese Pharmacopoeia, edited in 2015, Chapter 4, General Method 0931, the 2nd Method). An appropriate amount of the control sample was taken, weighed accurately, dissolved in dissolution medium and diluted quantitatively to prepare a solution containing about 10µg of the sample per 1ml, and then measured in the same way. The amount of dissolution per capsule was calculated.

The results were shown in Table 2 to Table 4.

**Table 2 Dissolution rate of the sodium salt hydrate of compound A in water**

| sodium salt hydrate (dissolution medium: purified water) | | | | | | |
|---|---|---|---|---|---|---|
| Time | 5min | 10min | 15min | 20min | 30min | 45min |
| 1 | 57.9 | 69.0 | 91.7 | 96.2 | 98.0 | 97.5 |
| 2 | 45.5 | 73.6 | 93.3 | 98.3 | 99.1 | 99.7 |
| 3 | 56.7 | 62.2 | 99.1 | 100.1 | 102.2 | 102.7 |
| 4 | 52.8 | 94.6 | 92.7 | 100.2 | 100.2 | 100.9 |
| 5 | 55.8 | 77.8 | 96.6 | 101.8 | 103.4 | 102.8 |
| 6 | 34.3 | 82.6 | 79.1 | 94.8 | 100.7 | 101.5 |
| Mean (%) | 50.5 | 76.6 | 92.1 | 98.6 | 100.6 | 100.8 |

**Table 3 Dissolution rate of the potassium salt hydrate of compound A in water**

| potassium salt hydrate (dissolution medium: purified water) | | | | | | |
|---|---|---|---|---|---|---|
| Time | 5min | 10min | 15min | 20min | 30min | 45min |
| 1 | 24.1 | 81.5 | 87.4 | 89.2 | 90.2 | 91.3 |
| 2 | 34.9 | 72.3 | 84.4 | 87.8 | 87.4 | 87.0 |
| 3 | 41.7 | 77.6 | 79.5 | 83.0 | 85.0 | 85.8 |
| 4 | 29.6 | 79.2 | 82.1 | 84.1 | 85.6 | 84.9 |
| 5 | 30.7 | 80.4 | 83.7 | 86.4 | 86.9 | 85.6 |
| 6 | 28.9 | 53.3 | 80.8 | 83.1 | 86.7 | 86.7 |
| Mean (%) | 31.6 | 74.1 | 83.0 | 85.6 | 87.0 | 86.9 |

**Table 4 Dissolution rate of the lithium salt hydrate of compound A in water**

| lithium salt hydrate (dissolution medium: purified water) | | | | | | |
|---|---|---|---|---|---|---|
| Time | 5min | 10min | 15min | 20min | 30min | 45min |
| 1 | 34.7 | 74.4 | 83.4 | 85.9 | 85.8 | 88.3 |
| 2 | 34.4 | 60.4 | 81.4 | 84.9 | 84.6 | 85.5 |
| 3 | 35.6 | 74.8 | 81.3 | 85.5 | 84.4 | 84.2 |
| 4 | 23.9 | 40.4 | 66.1 | 84.4 | 85.3 | 85.3 |
| 5 | 36.5 | 78.7 | 83.6 | 84.3 | 85.9 | 85.5 |
| 6 | 42.6 | 75.4 | 82.7 | 87 | 85.2 | 85.6 |
| Mean (%) | 34.6 | 67.4 | 79.8 | 85.3 | 85.2 | 85.7 |

The dissolution rate of compound A was tested by using the same method as described above, and the dissolution rate of compound A was basically zero due to its poor water solubility.

Based on the above contents, it can be known that all hydrates of three salts obtained in the present invention show good dissolution rate, and the sodium salt hydrate has the best dissolution rate.

### Test example 3: Stability Test

Test process: An appropriate amount of each test sample was placed on a clean watch glass without a lid and then placed under the conditions of light 45001x±5001x, high temperature of 60°C, high humidity of 92.5% RH for 5 days and 10 days, respectively. The properties and related substances were determined and the results were compared with the results at day 0 to observe the stability.

The test method for related substances was as follows.

Preparation of test sample solution: About 10 mg of each test sample was placed in a 10ml volumetric flask, and 50% acetonitrile aqueous solution was added to dissolve sample, and diluted to the mark, shaken well, and filtered to obtain the test sample solution. A precise amount of 10µl of the test sample solution was taken and injected according to the above chromatographic method, and the maximum single impurity and total impurity were calculated according to the area normalization method.

Test results:
Test results of influencing factors of the compound of formula A

| | | maximum single impurity% | total impurity% | property |
|---|---|---|---|---|
| Day 0 | | 0.08 | 0.32 | white powder |
| high temperature | Day 5 | 0.07 | 0.24 | white powder |
| | Day 10 | 0.08 | 0.25 | white powder |
| high humidity | Day 5 | 0.08 | 0.31 | white powder |
| | Day 10 | 0.07 | 0.26 | white powder |
| light condition | Day 5 | 4.66 | 5.28 | light yellow powder |
| | Day 10 | 15.02 | 15.25 | light yellow powder |

Test results of influencing factors for sodium salt hydrate

| | | maximum single impurity% | total impurity% | property |
|---|---|---|---|---|
| Day 0 | | 0.03 | 0.07 | white powder |
| high temperature | Day 5 | 0.02 | 0.07 | white powder |
| | Day 10 | 0.02 | 0.09 | white powder |
| high humidity | Day 5 | 0.02 | 0.07 | white powder |
| | Day 10 | 0.02 | 0.06 | white powder |
| light condition | Day 5 | 0.06 | 0.16 | light yellow powder |
| | Day 10 | 0.15 | 0.26 | light yellow powder |

Test results of influencing factors for potassium salt hydrate

| | | maximum single impurity% | total impurity% | property |
|---|---|---|---|---|
| Day 0 | | 0.34 | 0.49 | white powder |
| high temperature | Day 5 | 0.34 | 0.45 | white powder |
| | Day 10 | 0.33 | 0.45 | white powder |
| high humidity | Day 5 | 0.34 | 0.45 | Molten solid |
| | Day 10 | 0.31 | 0.48 | colorless liquid |
| light condition | Day 5 | 0.33 | 0.72 | light yellow powder |
| | Day 10 | 0.33 | 0.88 | light yellow powder |

Test results of influencing factors of lithium salt hydrate

| | | maximum single impurity% | total impurity% | character |
|---|---|---|---|---|
| Day 0 | | 0.59 | 0.87 | white powder |
| high temperature | Day 5 | 0.56 | 0.79 | white powder |
| | Day 10 | 0.56 | 0.78 | white powder |
| high humidity | Day 5 | 0.55 | 0.72 | white powder |
| | Day 10 | 0.56 | 0.76 | white powder |
| light condition | Day 5 | 0.61 | 0.93 | light yellow powder |
| | Day 10 | 0.78 | 1.21 | light yellow powder |

The above results show that the compound of formula A, and the hydrates of sodium salt, potassium salt and lithium salt obtained in the present application are relatively stable under high temperature and high humidity conditions. Under light condition, the compound of formula A degrades significantly, and the salt formation of the compound is significantly enhanced in light stability. It can be seen that the stability of sodium salt, lithium salt, and potassium salt is significantly better than that of the compound of formula A.

### Example 1

The sodium salt hydrate of the compound of formula A (CT-NA) prepared in Preparation Example 3 was used for the activity test. The test method was as follows.

### 1.1 Mice Treatment

Specific pathogen-free (SPF) female Balb/c mice (22-28 g; aged 8 weeks) were purchased from Shanghai SIPPR-BK Experimental Animal Co., Ltd. Mice were kept in an isolated ventilated cage (4-5 mice/cage) in an environment of 40-60% humidity, 24 ± 2°C, 12 h/12 h dark-light cycle and had free access to food and water.

### 1.2 Preparation of cigarette smoke (CS)-induced lung injury model and measurement of partial pressure of oxygen

Model preparation: The mice were randomly divided into 5 groups (12 mice in each group), which were the control group (the mice were exposed to fresh air), the saline group (the mice were exposed to cigarette smoke), dexamethasone (Dex) group (1mg/kg) (the mice were exposed to cigarette smoke), CT-NA 10mg/kg group and CT-NA 30mg/kg group (the mice were exposed to cigarette smoke). According to the grouping, the mice were intragastrically administered saline, Dex and CT-NA, respectively. Thereafter, the mice were exposed to fresh air or cigarette smoke generated from 3R4F research-grade cigarettes (containing about 600 mg TPM/m³ and 29.9 mg nicotine/m³) in a cuboid plastic box (65×50x×45 cm). Ten cigarettes were burned every day for seven days. After one cigarette was burned, the next cigarette was ignited. The daily body weight and general condition of the mice were checked. 24 hrs after the last exposure to the cigarette smoke, the partial pressure of oxygen (PO₂) of all mice was measured by the Moor VMS-OXY™ measuring instrument, which was used to measure the oxygenated/deoxygenated hemoglobin concentration and oxygen saturation (percentage) in the microcirculation at the wavelength range of 500 to 650 nm. Then, all mice were euthanized to collect bronchoalveolar lavage fluid (BALF) for measuring the total number of inflammatory cells, cytokine levels and albumin concentration. The lung tissue was collected for determination of lung weight coefficient, histological examination and MPO activity.

### 1.2.1 Inflammatory cells counting

After the mice were euthanized, the trachea was surgically exposed, and then the right lungs were lavaged three times with 0.4 mL/time of sterile normal saline containing 1% FBS and 5000 IU/L heparin to collect the BALF via tracheal tube. After measuring the total number of cells in BALF with a hemocytometer, the remaining BALF was centrifuged at 1000×g at 4°C for 10 minutes. The supernatant was aliquoted and stored at -80°C until the cytokine or albumin concentration was measured. The obtained cell pellets were coated on a glass slide. Then, according to the morphological standards of neutrophil, macrophage and lymphocyte, the smear was stained with Wright-Giemsa under an optical microscope to count 200 cells.

### 1.2.2 Lung weight ratio

As an index of pulmonary edema, the lung weight ratio was measured by dividing the individual lung weight of each mouse after aspirating the blood tissue from the lung surface, by the total body weight.

### 1.2.3 Albumin assay

The albumin concentration in the BALF supernatant was tested with albumin determination kits at 628nm by a spectrophotometer. The albumin concentration ratio assessed from BALF represented not only the albumin level but also the permeability of the pulmonary microvascular.

### 1.2.4 In vivo cytokine assay by ELISA

The expression levels of pro-inflammatory cytokines (TNF-a, IL-1β, IL-6), chemokines (KC) and anti-inflammatory cytokines (IL-10) in the BALF supernatant were determined using respective ELISA determination kits according to the instruction manual. After the optical density at 450 nm was measured, the expression of cytokines was calculated via the standard curve.

### 1.2.5 Pulmonary histopathology

The lower lobe of the left lung of each mouse was preserved in 10% neutral formalin for histopathological examination. The preserved lower lobes of the left lungs were taken out and embedded in paraffin, and then sectioned (4 um) to expose the maximum longitudinal view of the main intrapulmonary bronchus. The sections were stained with hematoxylin and eosin (H&E) by standard method. A 5-point scoring system was used to evaluate pulmonary edema, severity of inflammation, and infiltration of inflammatory cells. Briefly, the scoring system was as follows: 0 = normal; 1 = very mild; 2 = mild; 3 = moderate; 4 = marked; 5 = severe inflammation. Scoring was performed in at least three different fields for each lung section. The mean scores were derived from 12 mice.

### 1.2.6 MPO assay

In order to evaluate MPO activity, 50 mg strips of left lung tissue were washed, and then homogenized with normal saline. MPO activity was determined by measuring the changes in absorbance at 460nm according to the detection standard by using MPO assay kit.

### 1.2.7 Isolation of neutrophils and assessment of the effect of CT-NA on PGD₂-induced neutrophils migration

The mice were intragastrically administered glycogen (1.5%) at the dose range of 20ml/kg of body weight. 4hrs later, the mice were euthanized to isolate neutrophils from the peritoneal lavage. The effect of CT-NA on neutrophils migration was detected by using the Boyden chamber assay kit (3µm pore size). PGD₂ was used as a chemoattractant because the activated PGD₂/CRTH2 receptors promoted the migration of neutrophils. Initially, the isolated neutrophils (4×10⁵) were diluted in 100 µL HBSS and allowed to migrate toward PGD₂ (0.1, 1 and 10 µM) for 4 hrs to find out the suitable PGD₂ concentration. Then, the isolated neutrophils (4×10⁵) were pretreated with CT-NA (1 and 10 µM), and their migration to PGD₂ (1 µM) was evaluated by counting the migrated neutrophils. Moreover, another potent CRTH2 inhibitor OC459 was used to countercheck the outcomes of CT-NA.

### 1.2.8 Preparation of cigarette smoke extract (CSE)

The cigarette smoke generated from 3R4F research grade cigarettes was passed through 50ml PBS by a vacuum pump. Five cigarettes were used to make smoke that passed 50ml PBS, and each cigarette was lit for 5 minutes. The control solution was prepared in the absence of cigarettes by using a similar method. After extraction, CSE was stored at -80°C.

### 1.2.9 Isolation of primary macrophages and assessment of CSE-induced secretion of PGD₂ from primary macrophages

The primary macrophages were isolated from the peritoneal cavity, and the method was briefly described as follows. Thioglycolate (4%) was injected into the peritoneal cavity of mice at a dose of 20 ml/kg body weight for three consecutive days. On day 5 (48 hrs after the last thioglycolate injection), the mice were euthanized to isolate primary macrophages from the peritoneal lavage. The isolated primary macrophages (4×10⁵/well) were added to a 12-well plate and cultured at 37°C. Thereafter, the medium of the 12-well plate was replaced with a serum-free RPMI-1640 medium and incubated for 10-12 hrs, and then the primary macrophages were exposed to different concentrations of CSE (2%, 4%, and 8%) for 24 hrs. After the treatment was completed, the supernatant of the primary macrophages was harvested to measure the protein level of extracellularly secreted PGD₂ using ELISA kits according to the method of the instructions.

### 1.2.10 Cell viability assay

The cell lines of RAW 264.7 macrophages and mouse leukemic mononuclear macrophages were purchased from American Type Culture Collection (ATCC, Manassas, Virginia, USA). RAW 264.7 macrophage was cultured in RPMI-1640 medium containing penicillin (100U/ml), streptomycin (100µg/ml) and 10% FBS. The cytotoxicity of CT-NA (0-100µM) alone, and in a combination of PGD₂ (0-100µM) and CSE (1-10%) on RAW264.7 macrophage was assessed using methylthiazole-tetrazole (MTT) assay according to the manufacturer's protocol. Briefly, RAW 264.7 macrophages were seeded in a 96-well plate at a concentration of 4×10⁵ cells/ml for 24 hrs, and then exposed to CT-NA (0-100 µM) at 37° C for 1 hour. Next, RAW 264.7 macrophages were further exposed to CSE (4%) and PGD₂ (10 µM) for 24 hrs, and then treated with MTT (5 mg/ml) at 37°C for 4 hrs. Then, the supernatant of each well was replaced with DMSO (200 µl/well), and the absorbance at 570 nm was measured.

### 1.2.11 In vitro cytokines assay via ELISA and real-time polymerase chain reaction (RT-PCR)

RAW 264.7 macrophages were added to two 12-well plates. Thereafter, the medium of the 12-well plate was replaced with serum-free RPMI-1640 medium, and the macrophages were incubated for 10-12 hrs and then exposed to CT-NA (10 and 100 µM) for 1 hour. One hour later, one 12-well plate was treated with CSE (4%) and the other with PGD₂ (10 µM) for 24 hrs. After the treatment, the supernatant of the treated cells was collected to measure the protein levels of TNF-α, IL-1β, IL-6, KC and IL-10 extracellularly secreted using ELISA kits according to the method of the instructions. Subsequently, RNA samples from each treated plate were extracted and reverse-transcripted into cDNA with HiScript5xQRTSuperMix, and then subjected to RT-PCR. RT-PCR was performed with the BioRad CFX96 Touch™ Real-Time PCR Detection System (BioRad, USA) using AceQ^{®} qPCR SYBR Green Master Mix. And the threshold cycle numbers were obtained using BioRad CFX Manager Software. The primers used for RT-PCR reaction were shown in Table 1. β-actin was used as an internal control. The RT-PCR reactions were triplicated and the relative expression of target mRNA was normalized by the respective β-actin.

**Table 1**

| Genes | Primer sequences | Product | Amplification profile (temp. (°C)/time (*sec*.)) | | | Cycles |
|---|---|---|---|---|---|---|
| (Added sample) | (5' - 3') (bp) | length | denaturation | annealing | extension | (n) |
| IL-6 | F: TGCCTTCTTGGGACTGAT | 183 | 95/10 | 58/30 | 72/30 | 40 |
| (NM_031168) | R: TTGCCATTGCACAACTCTTT | | | | | |
| TNF-α | F: CCAGACCCTCACACTCAGAT | 187 | 95/10 | 58/30 | 72/30 | 40 |
| (NM_013693) | R: GACAAGGTACAACCCATCG | | | | | |
| IL-1β | F: GTTCCCATTAGACAACTGC | 199 | 95/10 | 58/30 | 72/30 | 40 |
| (NM_008361) | R: GATTCTTTCCTTTGAGGC | | | | | |
| KC | F:CAATGAGCTGCCCTGTCAGTG | 203 | 95/10 | 58/30 | 72/30 | 40 |
| (NM_011339) | R: CTTGGGGACACCTTTTAGCATC | | | | | |
| IL-10 | F: TCAAGGCGCATGTGAACTCC | 176 | 95/10 | 58/30 | 72/30 | 40 |
| (NM_010548.2) | R: GATGTCAAACTCACTCATGGCT | | | | | |
| β-actin | F: CACGATGGAGGGGCCGGACTCATC | 214 | 95/10 | 58/30 | 72/30 | 40 |
| (NM_007393) | R:TAAAGACCTCTATGCCAACACAGT | | | | | |

### 1.2.12 Statistical Analysis

Numerical data were expressed as means ± SEM. Statistical calculations were performed using SPSS (SPSS Inc., Chicago, IL). One-way ANOVA was applied to compare the F values. If p>0.05, Dunnett multiple comparisons test was used to calculate the difference of parametric data; if p<0.05, Mann-Whitney U non-parametric test was used to compare the difference. P<0.05 and p<0.01 were considered to be statistically significant.

### 2.1 Effect of CT-NA on CS-induced inflammatory cell number in BALF

24 hours after the last exposure to CS, the effect of CT-NA on the infiltration of total cells and various cells, especially neutrophils and macrophages, in BALF was analyzed by Wright-Giemsa staining method. As shown in Figures 10A and 10B, after CS exposure, the number of total cells, macrophages and neutrophils were increased significantly (p<0.01). Meanwhile, pretreatment with CT-NA (10 and 30 mg/kg) and Dex (1 mg/kg) significantly reduced the number of total cells, macrophages and neutrophils (p<0.01). These significant effects demonstrated that CT-NA could considerably ameliorate CS-induced pulmonary inflammation via CRTH2 antagonism.

### 2.2 Effect of CT-NA on CS-induced hypoxemia, pulmonary edema and lung permeability

CS-induced hypoxemia, pulmonary edema, and lung permeability were evaluated by measuring partial pressure of oxygen (PO₂), lung weight coefficient, and BALF albumin contents, respectively. In CS treatment group, PO₂ was evidently decreased (p<0.01), and the lung weight coefficient and BALF albumin content were significantly increased when compared with the control group (p<0.01), indicating that CS-induced animal models were successful. However, the CT-NA (10 and 30mg/kg) group significantly elevated the PO₂ (p<0.01) (Figure 10C), partially reduced the lung weight coefficient (p<0.05) (Figure 10D), and remarkably reduced the BALF albumin contents (p<0.01) (Figure 10E). These excellent results indicated that CRTH2 antagonism with CT-NA could effectively protect mice from CS-induced lung injury by mitigating hypoxemia, pulmonary permeability and edema.

### 2.3 Effect of CT-NA on CS-induced cytokines secretion in BALF

In order to determine whether CT-NA could affect the secretion of cytokines in BALF, the expression levels of pro-inflammatory cytokines (TNF-a, IL-1β, IL-6), chemokines (KC) and anti-inflammatory cytokine (IL-10) were detected using respective ELISA kits. As shown in Figure 11A, B, C and D, the expression levels of TNF-α, IL-1β, IL-6 and KC were significantly increased in the CS-exposed group compared to the control group (p<0.01). Meanwhile, CS-induced overexpression of TNF-α, IL-1β, IL-6 and KC were effectively reduced by CT-NA treatment (10 and 30 mg/kg) (p<0.01). In contrast, the expression level of IL-10 in the CS exposure group was significantly reduced (p<0.01), while CT-NA treatment reversed the CS-induced inhibition of IL-10 (p<0.01) (Figure 11E). These results indicated that blockade of CRTH2 receptors by CT-NA protected the CS-induced ALI mice from further pulmonary inflammation by inhibiting the production of pro-inflammatory cytokines and neutrophils chemokine and by stimulating the production of an anti-inflammatory cytokine (IL-10).

### 2.4 Effect of CT-NA on CS-induced pulmonary histopathologic alterations

In order to evaluate the protective effect of CT-NA on CS-induced pulmonary histopathologic changes, H&E staining experiments were performed. As shown in Figure 12A, the lung tissues of the control group showed normal pulmonary histology while CS-exposed lung tissues showed marked histopathological changes, such as infiltration of inflammatory cells , macrophages and neutrophils into alveolar cavity and interstitial edema. Conversely, these changes were strikingly improved by pretreatment with CT-NA (10 and 30 mg/kg) or Dex (1 mg/kg). In addition, pathological scores were also assessed to determine the severity of inflammation, infiltration of inflammatory cells and pulmonary edema. The results of lung inflammation scores showed that CS-exposure significantly increased mean pathological score (p<0.01) while CT-NA (10 and 30 mg/kg) and Dex (1 mg/kg) considerably reduced the mean pathological scores in a dose-dependent manner (p<0.01) (Figure 12B).The test results showed that CT-NA remarkably reduced the severity of CS-induced lung injuries by blocking CRTH2 receptor.

### 2.5 Effect of CT-NA on CS-induced MPO activity

Owing to the above-mentioned promising outcomes, MPO activity of lung tissues was further evaluated. MPO produced by activated neutrophils acts as an important marker of neutrophil infiltration and lung tissue damage. It was found that MPO activity of CS-exposed lung tissues was significantly increased as compared to fresh air-exposed (p<0.01) (Figure 13A). It was noticed that CT-NA (10 and 30mg/kg) and Dex (1mg/kg) attenuated the MPO activity (p<0.01), indicating that CRTH2 receptor blockade effectively inhibited the neutrophils infiltration into alveolar and interstitial spaces.

### 2.6. Effect of CT-NA on PGD₂-induced neutrophils migration in vitro

Prompted by the MPO test results, the direct effect of CT-NA on PGD₂-induced neutrophils migration was further assessed by using Boyden chamber assay kit, because the activated PGD₂/CRTH2 receptors promoted the migration and functions of neutrophils. Moreover, the inflammatory mediators released by neutrophils were mostly to exacerbate lung injury. Neutrophils isolated from the abdominal cavity of mice were challenged with 1.5% glycogen, and then subjected to Wright-Giemsa staining and cell viability assay to inspect the characteristics of neutrophils. Four hours of incubation showed significant migration of neutrophils (p<0.01) toward PGD₂ (1 and 10 µM) (Figure 13B). Meanwhile, pretreatment with CT-NA (1 and 10 µM) significantly attenuated PGD₂-induced neutrophils migration (p<0.01)

(Figure 13C). Likewise, OC459, another CRTH2 antagonist, also inhibited PGD₂-induced neutrophils migration (Figure 13C). Taken together, these data clearly showed that CRTH2 antagonists appreciably reduced PGD₂-induced neutrophils migration.

### 2.7 CSE promotes the secretion of PGD₂ from primary macrophages

In order to assess whether CSE affected the secretion of PGD₂, the isolated primary macrophages were treated with different concentrations of CSE (2%, 4% and 8%) for 24 hrs, and then the protein levels of extracellularly secreted PGD₂ were evaluated by PGD₂ ELISA kit. The treatment with CSE (4%) significantly promoted the secretion of PGD₂ as compared to the control group (p<0.01) (Figure 13D).

### 2.8 Effect of CT-NA on CSE- and PGD₂-induced cytokines secretion from RAW 264.7 macrophages

Based on the remarkable outcomes *in vivo,* it was further considered whether CT-NA treatment could inhibit the secretion of cytokines from CSE- and PGD₂-stimulated RAW 264.7 macrophages, because activated PGD₂/CRTH2 receptors on macrophages significantly increased disease severity via increased expression of pro-inflammatory cytokines. MTT assay showed that PGD₂ (10µM) plus CT-NA with a concentration of up to 100µM, and CSE 4% plus CT-NA with a concentration of up to 100µM were non-toxic to RAW 264.7 macrophages. Moreover, the results of ELISA (Figure 14) and RT-PCR (Figure 15) proved that CT-NA (10 and 100 µM) treatment not only inhibited the protein and mRNA levels of IL-1β, IL-6, TNF-α and KC produced by CSE- and PGD₂-stimulated RAW 264.7 macrophages (p<0.01), but also reversed CSE- and PGD₂-induced inhibition of IL-10 (p<0.01) in a dose-dependent manner. Therefore, the test results obtained *in vitro* (Figures 14 and 15) were similar to those obtained *in vivo* (Figure 11). Summing up, these data indicated that CRTH2 antagonism effectively improved the production of pro-inflammatory cytokines and chemokines and promoted the production of anti-inflammatory cytokines from CSE- and PGD₂-activated RAW 264.7 macrophages.

### Example 2

### 2.1.1 Mouse treatment, preparation of LPS-induced ALI model and measurement of oxygen saturation

Specific pathogen-free (SPF) Balb/c mice ( ♂ / ♀; 20-26 g; 8 weeks old) were purchased from Shanghai SIPPR-BK Experimental Animal Co., Ltd. Mice were kept in an isolated ventilated cage in an environment of 40-60% humidity, 24 ± 2 °C, and 12 hrs/12 hrs dark-light cycle, and had free access to food and water. The method for preparing the LPS-induced ALI model was simply described as follows. The mice were randomly divided into a control group (12 mice) and an LPS group (48 mice). The LPS group (48 mice) was further divided into four subgroups (12 mice in each subgroup). The unanaesthetized mice in the four subgroups of LPS were intragastrically administered with normal saline (NS), CT-NA at 10 mg/kg or 30 mg/kg, and Dex at 1 mg/kg, respectively. One hour later, the mice were anesthetized with sodium pentobarbital (intraperitoneal injection at 40 mg/kg), and then subjected to intratracheal instillation of NS to control group and LPS (4 mg/kg) to all LPS subgroups. 12 hrs later, the unanaesthetized mice of the LPS group were intragastrically administered with NS, CT-NA at 10 mg/kg or 30 mg/kg, or Dex at 1 mg/kg, respectively (Figure 16). Both normal saline and LPS were administered at 10 µl/10 g of body weight. 24 hrs after LPS challenge, the oxygen saturation (SO₂) of all mice was measured by a Moor VMS-OXY™ instrument which measured SO₂ (%) in the microcirculation at the wavelength range of 500 to 650 nm. SO₂ represented the percentage of oxygenated hemoglobin in the blood to total hemoglobin. After the measurement of SO₂, the BALF from each mouse was collected for inflammatory cell counting and classification, and for determination of albumin concentration and pro-inflammatory cytokine/chemokine levels, whereas the lung was also used for determination of the lung weight coefficient and of MPO activity as well as histological examination.

### 2.1.2 Cell counting and lung weight coefficient

The mice were euthanized to expose the trachea, and then the right lungs were lavaged three times with 0.4 mL/time of sterile normal saline containing bovine serum albumin (BSA) and 5000 IU/L heparin to collect the BALF via tracheal tube. After measuring the total cells in BALF with a hemocytometer, the remainder BALF was centrifuged at 1000×g at 4°C for 10 minutes. The supernatant was aliquoted and stored at -80°C until measurement of the pro-inflammatory cytokine or albumin concentration. The obtained cell pellets were smeared on glass slides. Then, the smears were stained with Wright-Giemsa to count 200 cells under an optical microscope according to the morphological criteria of neutrophils, macrophages and lymphocytes. The removed lung tissues were weighted after aspirating the surface blood, and the lung weight coefficient was calculated. The lung weight coefficient was an indicator of pulmonary edema, and it was measured by dividing the individual lung weight of each mouse by its total body weight.

### 2.1.3 Evaluation of pro-inflammatory cytokines and chemokines in vivo by ELISA

The expression levels of pro-inflammatory cytokines (TNF-a, IL-1β, IL-6), and chemokines (KC, mouse IL-8 homolog) in the BALF supernatant were determined by using respective ELISA determination kits according to the instruction manual. After measurements of the optical density at 450 nm, the expression level was calculated via the standard curves.

### 2.1.4 Histological examination

The lower lobe of the left lung of each mouse was inflated with neutral buffered 10% formalin instilled at room temperature under constant pressure of 22 to 25 cm H₂O for 48 hrs. The inflated lower lobes were taken out and embedded in paraffin, and then sectioned (4 um) to expose the maximum longitudinal view of the main intrapulmonary bronchus. Afterward, H&E staining was then used to assess pulmonary edema and infiltration of neutrophils and inflammatory cells under a light microscope. Pulmonary edema, hemorrhage, alveolar wall thickening and infiltration of neutrophils and inflammatory cells were counted and scored to evaluate the severity of lung injury. The scoring system was as follows: 0 = normal; 1 = very mild; 2 = mild; 3 = moderate; 4 = marked; 5 = severe. The total lung injury score was expressed as the sum of the four criteria. The mean scores were derived from 12 mice.

### 2.1.5 In vivo pulmonary vascular permeability assessment

Evans Blue was a dye that could quickly bind to albumin and was remains restricted within blood vessels because the endothelium was impermeable to albumin under normal physiological conditions. Pulmonary microvascular permeability was assessed by measuring the extravasation of Evans blue dye in the lungs. The method was briefly described as follows. The mice were randomly divided into control group (12 mice) and LPS group (48 mice).The LPS group (48 mice) was further divided into four subgroups (each subgroup contains 12 mice). To measure pulmonary microvascular permeability, the unanaesthetized mice of LPS group received intragastric instillation of NS, CT-NA at 10 mg/kg or 30 mg/kg, or Dex at 1 mg/kg respectively. One hour later, the anaesthetized mice were intratracheally instilled with NS (control group) and LPS (all LPS subgroups) at 10 µl/10 g of body weight. 24 hrs after LPS challenge, Evans blue dye (50 mg/kg) was injected into the caudal vein of all mice. 1 hour later, the mice were euthanized, then NS was slowly injected into the right ventricle in order to drain the blood from the lung tissue. The right lung was carefully removed,, sliced and placed in formamide (3ml/100mg) at room temperature. After 24 hrs of incubation, the samples were centrifuged at 500×g for 10 minutes (4°C). And then the absorbance of the Evans blue dye extracted in the supernatant was measured against formamide blank at 620 nm via standard curve method,and expressed as microgram of dye per 100 mg of wet lung weight. In addition, the concentration of albumin in BALF was measured at 628 nm using a spectrophotometer and an albumin measurement kit. The albumin concentration ratio assessed from BALF represented not only the albumin level but also the permeability of the pulmonary microvascular.

### 2.1.6 MPO activity assay

The MPO activity assay procedure was as follows. The strips of left lung tissue were accurately weigh and prepared into a 5% homogenate with a homogenization medium (the volume ratio of the left lung strip tissue and the homogenization medium was 1:19). Then the homogenate (0.9ml) and reaction buffer (0.1ml) were sufficiently mixed at a ratio of 9:1 (if there was not enough homogenate, the volumns of 5% tissue homogenate and reaction buffer could be reduced accordingly at the ratio of 9:1), and then incubated at 37°C for 15 minutes. Then MPO activity was determined by measuring the changes in absorbance at 460nm via the standard curves by using MPO assay kits.

### 2.1.7 Isolation of neutrophils and evaluation of PGD₂-induced neutrophils migration in vitro

The method for isolating neutrophils and testing effect of CT-NA on neutrophils migration was briefly described as follows. 1.5% glycogen was injected intragastrically into mice at a dose of 20 ml/kg body weight. 4 hrs later, the mice were euthanized to isolate neutrophils from the peritoneal lavage. Effect of CT-NA on the migration of neutrophils was detected using the Boyden chamber assay kit (3um pore size, Billerica, MA). PGD₂ was used as a chemoattractant because the activated PGD₂/CRTH2 receptors promoted the neutrophils migration. Firstly, the isolated neutrophils were inoculated at 4×10⁵ cells/ml into the upper side of well of the Boyden chamber, and the lower chamber contained different concentrations of PGD₂ (0.1, 1 and 10µM), and the neutrophils were allowed to migrate towards PGD₂ for 4 hrs at 37°C in order to find out the suitable PGD₂ concentration. After evaluation of the suitable PGD₂ concentration, the migration of neutrophils pretreated with CT-NA (1 and 10 µM) and OC459 (10 µM), another effective CRTH2 inhibitor, toward PGD₂ (1 µM) was assessed for 4 hrs.

### 2.1.8 Isolation of peritoneal macrophages and assessment of LPS-induced secretion of PGD₂ from peritoneal macrophages

The method was briefly described as follows. Thioglycolate (4%) was intraperitoneally injected to mice at a dose of 20 ml/kg of body weight for three consecutive days. 48 h after the last thioglycolate injection (on day 5), the peritoneal macrophages were isolated from the peritoneal lavage of the euthanized mice. The isolated peritoneal macrophages were added to a 12-well plate (4×10⁵/well) and cultured at 37°C. Non-adherent cells were removed by gently washing three times with warm PBS. At this time, more than 90% of the cells were macrophages, which were cultured in DMEM/high glucose medium containing penicillin (100 U/ml), streptomycin (100 µg/ml) and 10% FBS at 37°C. After acclimation, serum-free DMEM/high glucose was added to a 12-well plate for 10-12 hrs, and then treated with different concentrations of LPS (0.01, 0.1, 1 and 10 µM) for 24 hrs. Afterward, the supernatant of the peritoneal macrophages was collected to measure the protein level of PGD₂ using ELISA kits according to the method of the instructions.

### 2.1.9 Cell culture and cell viability assay

The cell lines of RAW 264.7 macrophages and mouse leukemia mononuclear macrophages were purchased from ATCC (Manassas, Virginia), and cultured in RPMI-1640 medium containing penicillin (100U/ml), streptomycin (100µg/ml) and 10% fetal bovine serum. RAW264.7 macrophage is an excellent model for screening anti-inflammatory drugs and evaluating the inhibitor pathways that stimulated the production of pro-inflammatory cytokines and enzymes. MTT was used to determine the cytotoxicity of CT-NA alone and its combination with PGD₂ and LPS on RAW264.7 macrophages and isolated peritoneal macrophages according to the manufacturer's protocol. Briefly, RAW 264.7 macrophages were plated in a 96-well plate at a concentration of 4×10⁵ cells/ml for 12 hrs, and then exposed to CT-NA (0-200 µM) at 37° C for 1 hour. Next, RAW 264.7 macrophages were further exposed to LPS (100ng/ml) and PGD₂ (10µM) for 24 hrs, and then treated with MTT (5mg/ml) at 37°C for 4 hrs. Then, the supernatant of each well was replaced with DMSO (200 µl/well), and the absorbance was measured at 570 nm.

### 2.1.10 In vitro ELISA assay and real-time polymerase chain reaction (RT-PCR) analysis

For ELISA and RT-PCR, RAW 264.7 macrophages were acclimated to two 12-well plates at 70-80% confluence. Thereafter, the medium in the 12-well plate was replaced with serum-free RPMI-1640 medium for 10-12 hrs and then exposed to CT-NA (10 and 100 µM) for 1 hour. One hour later, one 12-well plate was treated with LPS (100ng/ml) for 24 hrs, and the other was treated with PGD₂ (10µM) for 24 hrs. After the treatment, the supernatant of the treated cells was collected to measure the protein levels of TNF-α, IL-1β, IL-6, and KC using ELISA kits according to the method of the instructions. Subsequently, RNA samples from each treated plate were extracted and reverse-transcripted into cDNA with HiScript5×QRTSuperMix, and then subjected to RT-PCR using BioRad CFX96 Touch™ real-time PCR detection system (San Diego, California). Similarly, total RNAs extracted from treated isolated peritoneal macrophages were subjected to analyze the mRNA levels of IL-1β, TNF-α, IL-6 and KC. The primers used in the RT-PCR reaction were shown in Table 2. β-actin was used as an internal control. The RT-PCR reactions were triplicated and the relative expression of target mRNA was normalized by the respective β-actin.

**Table 2**

| Gene | primer sequence **(5' ― 3')** | Product length (bp) |
|---|---|---|
| **IL-6** | F: TGCCTTCTTGGGACTGAT | 183 |
| | R: TTGCCATTGCACAACTCTTT | |
| **TNF-α** | F: CCAGACCCTCACACTCAGAT | 187 |
| | R: GACAAGGTACAACCCATCG | |
| **IL-1β** | F: GTTCCCATTAGACAACTGC | 199 |
| | R: GATTCTTTCCTTTGAGGC | |
| **KC** | F:CAATGAGCTGCGCTGTCAGTG | 203 |
| | R: CTTGGGGACACCTTTTAGCATC | |
| **β-actin** | F: CACGATGGAGGGGCCGGACTCATC | 214 |
| | R: TAAAGACCTCTATGCCAACACAGT | |

### 2.1.11 Western Blotting Analysis

The method for total protein extraction and Western blotting determination was as follows. The lung tissue was homogenized in RIPA buffer (0.5M Tris-HCl, pH 7.4, 1.5M NaCl, 2.5% deoxycholic acid, 10% NP-40, 10mM EDTA) containing protease and phosphatase inhibitors (Sigma-Aldrich, St. Louis, MO). RAW264.7 macrophages were seeded into two 6-well plates at 70-80% confluence. After overnight starvation with serum-free RPMI-1640 medium, RAW264.7 macrophages were pretreated with CT-NA (0.5, 1, 10 and 100 µM) for 1 hour. Thereafter, one 6-well plate was treated with LPS (100ng/ml) for 1 hour, and the other was treated with PGD₂ (10µM) for 1 hour. After washing three times with PBS, the cells were directly lysed for 30 minutes with shaking in RIPA buffer containing protease and phosphatase inhibitors in an ice environment. Then, the lysate was centrifuged at 12,300×g for 15 minutes at 4°C, and the supernatant was collected. Bradford Protein Assay (BCA) was performed to measure protein concentration. Equal amounts of protein (30 µg) were resolved on 12% SDS-PAGE and transferred to 0.45 um polyvinylidene fluoride (PVDF) membranes (Millipore, Bedford, MA). The membranes were blocked with 5% (wt/vol) skimmed milk powder at room temperature for 1-2 hrs to reduce non-specific binding. Then the membranes were incubated overnight with the primary antibodies specific to IκBα (1:1000), phospho-IκBα (1:1000), NE-κBP65 (1:1000), phosphorylated NF-κBP65 (1:1000) at 4°C, and incubated with the secondary antibodies IRDye680 and 800 at room temperature for 1 hour, followed by washing with TBST three times. The Odyssey infrared imaging system (LI-COR Biosciences Lincoln, NE) was used to visualize the immune response signals. Western blotting assays were triplicated, and β-actin was used as an internal standard. The same procedure was performed for the lung tissues after homogenization.

### 2.1.12 Statistical Analysis

Numerical data were expressed as means ± SEM. Statistical calculations were performed using SPSS (SPSS Inc., Chicago, IL). One-way ANOVA was applied to compare the F values. If p>0.05, Dunnett multiple comparisons test was used to calculate the difference of parametric data; if p<0.05, Mann-Whitney U non-parametric test was used to compare the difference. P<0.05 and p<0.01 were considered to be statistically significant.

### 3.11 CT-NA alleviates LPS-induced lung injury

24 hrs after LPS challenge, BALF was collected to analyze the effect of CT-NA on cell infiltration by Wright-Giemsa staining method. The LPS challenge enhanced the infiltration of total cells, neutrophils and macrophages when compared with the NS challenge (P<0.01). However, CT-NA at 10 and 30 mg/kg significantly and dose-dependently attenuated the LPS-induced increase of total cells, neutrophils and macrophages but not lymphocytes in BALF when compared with the vehicle-treated control group (P<0.01). CT-NA at a dose of 30 mg/kg showed essentially same performance as that of 1 mg/kg Dex (Figure 17A). The LPS-induced hypoxemia and pulmonary edema were evaluated by measuring SO₂ and lung wet weight coefficient, respectively, revealed a lower SO₂ and a higher lung wet weight coefficient in the LPS-treated group than in the control group (P<0.01), and treatment with CT-NA at 10 or 30 mg/kg significantly increased SO₂ (P<0.01) and notably reduced the lung wet weight coefficient (P<0.01) in a dose-dependent manner (Figures 17B and C). CT-NA at 10 or 30mg/kg and Dex at 1mg/kg also enhanced SO₂ and reduced lung wet weight coefficient respectively. The above results indicated that CRTH2 antagonism by CT-NA could significantly improve lung inflammation, hypoxemia and pulmonary edema induced by LPS in ALI models.

### 3.2 CT-NA improved LPS-induced pro-inflammatory cytokines and chemokines production in BALF

The expression levels of IL-1β, TNF-α, IL-6 and KC in the collected BALF were measured by using respective ELISA kits to determine the effects of CT-NA on the production of pro-inflammatory cytokines and chemokines. LPS challenge significantly increased the expression of IL-1β, TNF-α, IL-6 and KC when compared with the vehicle challenge group (P<0.01). On the contrary, CT-NA at 10 or 30 mg/kg and Dex at 1 mg/kg effectively reduced the production of IL-1β, TNF-α, IL-6 and KC in a dose-dependent manner (P<0.05 or P<0.01) (Figures 18A-D). These data suggested that antagonism to CRTH2 by CT-NA could protect LPS-induced ALI mice from further lung inflammation caused by pro-inflammatory cytokines and neutrophil chemokines.

### 3.3 CT-NA attenuated LPS-induced pulmonary histopathologic alterations

The protective effect of CT-NA against LPS-induced pulmonary histopathology alterations was examined in H&E stained and paraffin-embedded pulmonary sections. LPS-challenged Mice showed significant histopathological changes compared with control mice (Figure 19A). In contrast, LPS-challenged mice treated with CT-NA at 10 or 30 mg/kg or Dex at 1 mg/kg showed significant reduction in these histopathological changes, and the effect of CT-NA on histopathological changes showed a dose dependence (Figure 19A). As compared with vehicle challenge, LPS challenge significantly increased the mean pathological scores, in terms of bleeding and infiltration of inflammatory cells and neutrophils into peribronchiolar and perivascular tissues (P<0.01), but CT-NA at doses of 10 mg/kg (P<0.05) and 30mg/kg (P<0.01) or Dex at a dose of 1 mg/kg (P<0.01) considerably reduced the pathological scores, and the results of CT-NA showed a dose-dependence again (Figure 19B). Therefore, blockage of the CRTH2 receptor by CT-NA significantly reduced the severity of LPS-induced pulmonary injuries and reversed the impairments of lung tissue.

### 3.4 CT-NA minimized pulmonary vascular permeability

The protective effect of CT-NA on LPS-induced pulmonary vascular permeability was determined by measuring the albumin content in BALF and the extraversion of Evans blue dye into the lungs. The albumin content in BALF was significantly increased in the LPS-challenged groups compared to the control group (P<0.01), while CT-NA at 10mg/kg (P<0.05) and 30mg/kg (P<0.01) or Dex at 1mg/kg (P<0.01) considerably reduced the albumin content in BALF (Figure 20A). The pulmonary vascular leakage and quantitative extravasation of Evans blue dye were significantly higher in the LPS-induced group than in the control group (P<0.01), and administration of CT-NA at 10mg/kg (P<0.05) or 30mg/kg (P<0.01) or Dex at 1mg/kg (P<0.01) significantly reduced LPS-induced pulmonary vascular leakage and extravasation of Evans Blue dye (Figure 20B). Therefore, these results indicated that CRTH2 antagonism could effectively improve the pulmonary vascular permeability of LPS-induced ALI mice.

### 3.5 CT-NA reduced LPS-induced pulmonary MPO activity

MPO was produced by activated neutrophils and was an important marker of neutrophils infiltration and pulmonary tissue injury. The increase in MPO activity reflected the increased accumulation of activated neutrophils in the lung. The MPO activity was significantly higher in the lung tissues from LPS-challenged group than from the control group (P<0.01). CT-NA treatment at 10mg/kg (P<0.01) or 30mg/kg (P<0.01) or Dex treatment at 1mg/kg (P<0.01) remarkably reduced MPO activity, with CT-NA again showing a dose dependence (Figure 21A). Therefore, CRTH2 receptor blockade with CT-NA could effectively inhibit the neutrophils infiltration into the alveolar and interstitial spaces..

### 3.6 CT-NA attenuated PGD₂- induced neutrophils migration in vitro

The effect of CT-NA on the migration of neutrophils was evaluated via the transwell assay, because the harmful inflammatory mediators released by neutrophils were the main causes of ALI symptoms. The characteristics of isolated neutrophils were evaluated by Wright-Giemsa staining and cell viability determinations were performed. The activated PGD₂/CRTH2 receptors promoted the migration and function of neutrophils, so PGD₂ was used as a chemoattractant. After 4 hrs of incubation, significant migration of neutrophils towards PGD₂ with a concentration ranging from 1 to 10 µM (P<0.05 or P<0.01) was observed (Figure 21B). Pretreatment of neutrophils with CT-NA at 1µM (P<0.01) or 10 µM (P<0.01) or OC459 at 10 µM (P<0.01) significantly reduced the neutrophils migration induced by 1 µM PGD₂ (Figure 21C). Summing up, these data indicated that CRTH2 antagonists significantly attenuated the neutrophils migration toward PGD₂.

### 3.7 LPS promoted the secretion of PGD₂ from isolated peritoneal macrophages

The isolated peritoneal macrophages were treated with different concentrations of LPS to evaluate the amounts of extracellularly secreted PGD₂ protein by ELISA kits. Treatment with LPS at a concentration of 0.01-10 µM (all P<0.01) significantly promoted the secretion of PGD₂ compared to vehicle treatment (Figure 21D).

### 3.8 CT-NA reduced the secretion of pro-inflammatory cytokines and chemokines from RAW264.7 macrophages and isolated peritoneal macrophages induced by LPS and PGD₂

The activation of PGD₂/CRTH2 receptors on macrophages could significantly worsen the disease condition by over-expression of pro-inflammatory cytokines that served as key factors in the pathogenesis of ALI.. Therefore, the effect of CT-NA treatment on either LPS- or PGD₂-induced production of pro-inflammatory factors was also examined in macrophages. MTT assay showed that LPS (100ng/ml) plus CT-NA up to 100µM or PGD₂ (10µM) plus CT-NA up to 100µM were non-toxic to RAW264.7 macrophages or isolated peritoneal macrophages. The effects of CT-NA on the expression of pro-inflammatory cytokines and chemokines were measured by ELISA. CT-NA at doses of 10 and 100µM inhibited the protein expression of IL-1β, TNF-α, IL-6 and KC in response to LPS (Figures 22A-D) or PGD₂ (Figures 22E-H) stimulation in a dose-dependent manner, respectively. Quantitative RT-PCR proved that CT-NA at doses of 10 and 100 µM reduced mRNA expressions of IL-1β, TNF-α, IL-6 and KC (all P<0.05 or all P<0.01) in LPS- or PGD₂-stimulated RAW264.7 macrophages (Figures 23A-H) and isolated peritoneal macrophages (Figures 24A-H) in a dose-dependent manner. Therefore, these *in vitro* results were consistent with those *in vivo* as shown in Figure 19, indicating that the CRTH2 antagonism with CT-NA effectively improved the production of pro-inflammatory cytokines and chemokines from LPS- or PGD₂-induced RAW264.7 macrophages and isolated peritoneal macrophages.

### 3.9 CT-NA inhibited P65 activation in vitro and in vivo

The underlying mechanism by which CT-NA significantly inhibited LPS-induced ALI was explored by Western blotting analysis. The study was focused on the effect of CT-NA on the LPS-induced NF-κB activation pathway, because NF-κB was necessary for the increases in pulmonary vascular permeability, neutrophils infiltration and activation of pro-inflammatory mediators. The treatment of RAW264.7 macrophages and pulmonary tissues with LPS strongly induced phosphorylation and degradation of IκBα, thereby increasing or decreasing phosphorylated P65 or P65 levels, respectively, when compared to the treatment with vehicle (Figures 25A and B). Treatments with CT-NA of RAW264.7 macrophages at 0.5, 1.0, 10, or 100 µM, or of ALI mice at 10 mg/kg or 30 mg/kg, reduced LPS-induced IκBα and P65 activation in a dose-dependent manner, and strongly reversed the degradation of IκBα and P65 induced by LPS (Figures 25A and B). Therefore, the CRTH2 receptor antagonist CT-NA protected mice from LPS-induced ALI most probably by inhibiting NF-κB signaling.

In summary, the experimental results showed that the heterocyclic compound represented by formula A, the hydrate thereof, the pharmaceutically acceptable salt thereof (such as alkali metal salt) or the hydrate of said salt (such as alkali metal salt) strikingly alleviated the acute lung injury induced by cigarette smoke or LPS through inhibition of inappropriate pulmonary migration of macrophages and neutrophils, reduction of pulmonary vascular permeability, amelioration of pro-inflammatory cytokines and chemokines production, and argumentation of IL-10 production. Moreover, the crystal form of the heterocyclic compound represented by formula A, the crystal form of the hydrate thereof, the pharmaceutically acceptable alkali metal salt thereof or the hydrate of said alkali metal salt had a superior therapeutic or alleviating effect on acute respiratory distress syndrome through attenuation of blockade or alleviation of inflammatory lung injuries, reduction of pulmonary edema, and maintainance of tissue oxygen supply.

The embodiments of the present invention have been described above. However, the present invention is not limited to the above-mentioned embodiment. Any modification, equivalent replacement, improvement, etc. made within the spirit and principle of the present invention should be included in the protection scope of the present invention.

## Claims

1. A pharmaceutically acceptable salt of a heterocyclic compound represented by formula A or a hydrate thereof, wherein the pharmaceutically acceptable salt is selected from alkali metal salt, and preferably sodium salt, lithium salt or potassium salt,

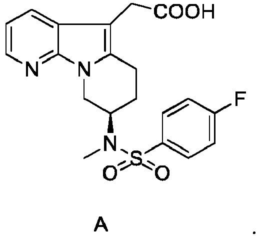

2. The pharmaceutically acceptable salt of a heterocyclic compound represented by formula A or a hydrate thereof of claim 1, wherein the hydrate is a monohydrate;
preferably, the hydrate is selected from a compound represented by the following formula A-N, A-K or A-L: 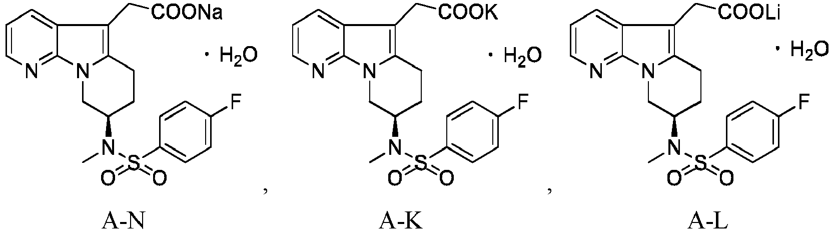
preferably, the compound represented by formula A-N is a crystalline hydrate, which has characteristic peaks at 2θ angles of 16.4±0.2°, 18.9±0.2°, 21.7±0.2°, and 24.0±0.2° in the X-ray powder diffraction spectrum using Cu-Ka radiation;
preferably, the crystalline hydrate has characteristic peaks at 2θ angles of 11.8±0.2°, 16.4±0.2°, 16.7±0.2°, 16.9±0.2°, 17.1±0.2°, 17.8±0.2°, 18.6±0.2°, 18.9±0.2°, 21.7±0.2°, 23.7±0.2°, and 24.0±0.2° in the X-ray powder diffraction spectrum using Cu-Ka radiation;
more preferably, the crystalline hydrate has characteristic peaks at 2θ angles of 5.6±0.2°, 11.8±0.2°, 14.0±0.2°, 15.8±0.2°, 16.4±0.2°, 16.7±0.2°, 16.9±0.2°, 17.1±0.2°, 17.8±0.2°, 18.6±0.2°, 18.9±0.2°, 20.3±0.2°, 21.7 ± 0.2°, 23.7±0.2°, 24.0±0.2°, 26.1±0.2°, 28.1±0.2°, 28.5±0.2°, and 29.8±0.2° in the X-ray powder diffraction spectrum using Cu-Ka radiation;
further preferably, the crystalline hydrate has an X-ray powder diffraction spectrum substantially as shown in FIG. 4 using Cu-Ka radiation;
preferably, the potassium salt compound represented by formula A-K is a crystalline hydrate, which has characteristic peaks at 2θ angles of 11.7±0.2°, 15.6±0.2°, 16.6±0.2°, 17.9±.2°, 18.5±0.2°, 21.4±0.2°, 24.0±0.2°, and 28.2±0.2° in the X-ray powder diffraction spectrum using Cu-Ka radiation;
more preferably, the crystalline hydrate of potassium salt has characteristic peaks at 2θ angles of 11.7±0.2°, 15.6±0.2°, 15.9±0.2°, 16.6±0.2°, 17.4±0.2°, 17.9±0.2°, 18.5±0.2, 21.4±0.2°, 23.5±0.2°, 24.0±0.2°, 27.7±0.2°, and 28.2±0.2° in the X-ray powder diffraction spectrum using Cu-Ka radiation;
more preferably, the crystalline hydrate of potassium salt has characteristic peaks at 2θ angles of 11.7±0.2°, 14.0±0.2°, 15.6±0.2°, 15.9±0.2°, 16.6±0.2°, 17.4±0.2°, 17.9±0.2°, 18.5±0.2°, 20.1±0.2°, 21.4±0.2°, 23.5±0.2°, 24.0±0.2°, 27.5±0.2°, 27.7±0.2°, 28.2±0.2°, 28.6±0.2°, 29.3±0.2°, and 29.6±0.2° in the X-ray powder diffraction spectrum using Cu-Ka radiation;
further preferably, the crystalline hydrate of potassium salt has an X-ray powder diffraction spectrum substantially as shown in FIG. 6;
preferably, the compound represented by formula A-L is a crystalline hydrate, which has characteristic peaks at 2θ angles of 16.7±0.2°, 18.8±0.2°, 21.9±0.2°, and 23.9±0.2° in the X-ray powder diffraction spectrum using Cu-Ka radiation;
more preferably, the crystalline hydrate of lithium salt has characteristic peaks at 2θ angles of 5.6±0.2°, 8.8±0.2°, 11.8±0.2°, 14.0±0.2°, 16.3±0.2°, 16.7±0.2°, 16.9±0.2°, 17.0±0.2°, 17.7±0.2°, 18.5±0.2°, 18.8±0.2°, 21.9±0.2°, 23.9±0.2°, and 28.2±0.2° in the X-ray powder diffraction spectrum using Cu-Ka radiation;
further preferably, the crystalline hydrate of lithium salt has an X-ray powder diffraction spectrum substantially as shown in FIG. 8.

3. A method for preparing a pharmaceutically acceptable salt of a heterocyclic compound represented by formula A or a hydrate thereof of claim 1 or 2, which comprises the following steps:
dissolving the compound represented by formula A in a ketone solvent; adding an aqueous solution of alkali metal hydroxide into the ketone solvent for reaction; and then filtering and drying to obtain it; wherein the alkali metal hydroxide is sodium hydroxide, lithium hydroxide or potassium hydroxide.

4. A pharmaceutical composition comprising a pharmaceutically acceptable salt of a heterocyclic compound represented by formula A or a hydrate thereof of claim 1 or 2.

5. A pharmaceutical composition for treatment of acute lung injury or acute respiratory distress syndrome, comprising a therapeutically effective amount of a heterocyclic compound represented by formula A, a pharmaceutically acceptable salt or a hydrate thereof;
preferably, the pharmaceutical composition further comprises at least one pharmaceutically acceptable carrier;
preferably, the carrier is an inert, non-toxic excipient, vehicle or diluent, for example, the carrier is selected from one, two or more of the following group consisting of disintegrant, glidant, lubricant, filler, adhesive, coloring agent, effervescent agent, flavoring agent, preservative, and coating material.

6. A use of a heterocyclic compound represented by formula A in preparing a medicament for treatment or prevention of a CRTH2-mediated disease; 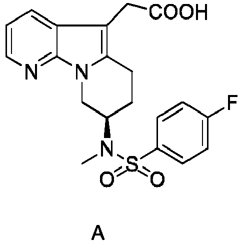
preferably, the heterocyclic compound represented by formula A is selected from the pharmaceutically acceptable salt thereof or the hydrate of said salt;
still preferably, the pharmaceutically acceptable salt of the heterocyclic compound represented by formula A or the hydrate of the salt has the definition as defined in claim 1 or 2;
preferably, the CRTH2-mediated disease includes but is not limited to allergic rhinitis, nasal congestion, runny nose, perennial rhinitis, rhinitis, asthma including allergic asthma, chronic obstructive pulmonary disease and other forms of pneumonia; sleep sickness and sleep-wake cycle disorder; dysmenorrhea and premature birth related to smooth muscle contraction induced by prostaglandin; eosinophil-related disease; thrombosis; glaucoma and vision disease; obliterative vascular disease; congestive heart failure; disease or condition that requires anticoagulant therapy, such as post-injury treatment or post-operative treatment; inflammation; gangrene; Raynaud's disease; mucus secretion disorder including cell protection; pain and migraine; disease that requires control of bone formation and resorption, such as osteoporosis; shock; heat regulation including fever; and immune disease or disorder that requires immune regulation,
more preferably, the CRTH2-mediated disease is selected from allergic rhinitis, pulmonary congestion and asthma including allergic asthma.

7. A use of at least one of a heterocyclic compound represented by formula A, a pharmaceutically acceptable salt thereof or a hydrate thereof in preparing a medicament for treatment of acute lung injury or acute respiratory distress syndrome,

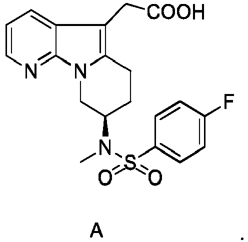

8. The use of claim 7, wherein the acute lung injury is selected from cigarette smoke (CS)-induced or lipopolysaccharide (LPS)-induced acute lung injury.

9. The use of claim 6 or 7, wherein the pharmaceutically acceptable salt of the heterocyclic compound represented by formula A is selected from alkali metal salts;
preferably, the hydrate is selected from the hydrate of the heterocyclic compound represented by formula A or the hydrate of the alkali metal salt of the heterocyclic compound represented by formula A;
preferably, the heterocyclic compound represented by formula A or the pharmaceutically acceptable salt thereof is in a crystalline form, such as a crystal form of the heterocyclic compound represented by formula A, a crystal form of hydrate thereof, a crystal form of the pharmaceutically acceptable alkali metal salt or a crystal form of the alkali metal salt hydrate;
preferably, the crystalline form of the heterocyclic compound represented by formula A or the hydrate thereof has characteristic peaks at 2θ angles of 11.1±0.2°, 11.4±0.2°, 17.9±0.2°, 22.6±0.2°, and 24.4±0.2° in the X-ray powder diffraction spectrum using Cu-Ka radiation;
preferably, the crystalline form has characteristic peaks at 2θ angles of 8.6±0.2°, 11.1±0.2°, 11.4±0.2°, 14.1±0.2°, 16.1±0.2°, 17.9±0.2°, 20.9±0.2°, 22.6±0.2°, 24.4±0.2°, and 25.8±0.2° in the X-ray powder diffraction spectrum using Cu-Ka radiation;
more preferably, the crystalline form has characteristic peaks at 2θ angles of 8.6±0.2°, 11.1±0.2°, 11.4±0.2°, 14.1±0.2°, 15.6±0.2°, 16.1±0.2°, 17.9±0.2°, 18.3±0.2°, 20.9±0.2°, 22.6±0.2°, 24.4±0.2°, 25.8±0.2°, 26.5±0.2°, and 28.9±0.2° in the X-ray powder diffraction spectrum using Cu-Ka radiation;
further preferably, the crystal form has an X-ray powder diffraction spectrum substantially as shown in FIG. 1;
preferably, the crystal form of the hydrate of the heterocyclic compound represented by formula A is a monohydrate;
still preferably, the crystal form is a single crystal having the following single crystal parameters:
| | |
|---|---|
| C₂₀ H₂₂ F N₃ O₅ S | *V*=3937.1(13)Å³ |
| *Mr*=435.47 | Z=8 |
| monoclinic crystal system, C2 | D*ₓ*=1.469mg/m³ |
| *a* = 17.022(3)Å | Mo *K*α radiation (λ = 0.71073_) |
| *b* = 11.380(2)Å | *θ*=2.20°-28.0° |
| c = 21.738(4)Å | *µ*=0.213mm⁻¹ |
| *α*=90° | *T*=153(2)k |
| *β*=110.783(2))° | bulk, colorless |
| *γ*=90° | 0.50×0.34×0.21mm |
preferably, the pharmaceutically acceptable alkali metal salt thereof has the definition as defined in any one of claims 1-3.

10. The use of any one of claims 6-8, wherein the pharmaceutically acceptable alkali metal salt of the heterocyclic compound represented by formula A is a sodium salt, a lithium salt or a potassium salt; the hydrate of the alkali metal salt is selected from the hydrate of sodium salt, lithium salt or potassium salt;
preferably, the crystal form of the alkali metal salt of the compound of formula A is in the form of a hydrate;
more preferably, the crystalline hydrate of the alkali metal salt of the compound of formula A is a monohydrate;
preferably, the crystalline hydrate of the alkali metal salt of the compound of formula A is selected from a compound represented by the following formulas A-N, A-L or A-K: 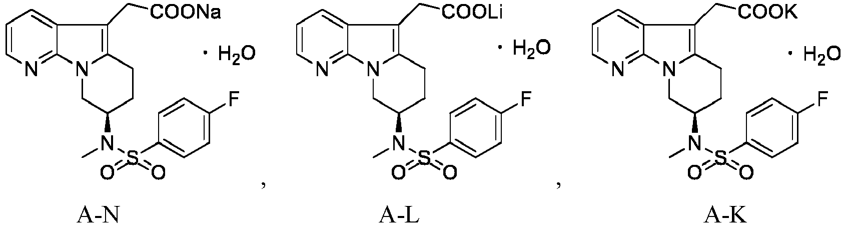
preferably, the compound represented by formula A-N is a crystalline hydrate, which has characteristic peaks at 2θ angles of 16.4±0.2°, 18.9±0.2°, 21.7±0.2°, and 24.0±0.2° in the X-ray powder diffraction spectrum using Cu-Ka radiation;
preferably, it has characteristic peaks at 2θ angles of 11.8±0.2°, 16.4±0.2°, 16.7±0.2°, 16.9±0.2°, 17.1±0.2°, 17.8±0.2°, 18.6±0.2°, 18.9±0.2°, 21.7±0.2°, 23.7±0.2°, and 24.0±0.2° in the X-ray powder diffraction spectrum using Cu-Ka radiation;
more preferably, the crystalline hydrate has characteristic peaks at 2θ angles of 5.6±0.2°, 11.8±0.2°, 14.0±0.2°, 15.8±0.2°, 16.4±0.2°, 16.7±0.2°, 16.9±0.2°, 17.1±0.2°, 17.8±0.2°, 18.6±0.2°, 18.9±0.2°, 20.3±0.2°, 21.7 ± 0.2°, 23.7±0.2°, 24.0±0.2°, 26.1±0.2°, 28.1±0.2°, 28.5±0.2°, and 29.8±0.2° in the X-ray powder diffraction spectrum using Cu-Ka radiation;
further preferably, the crystalline hydrate has an X-ray powder diffraction spectrum (XRPD) substantially as shown in FIG. 4;
preferably, the mass fraction of water in the crystal hydrate of the compound represented by formula A-N is 3.4-4.4%;
more preferably, the potassium salt compound represented by formula A-K is a crystalline hydrate, which has characteristic peaks at 2θ angles of 15.6±0.2°, 21.4±0.2°, and 24.0±0.2° in the X-ray powder diffraction spectrum using Cu-Ka radiation;
preferably, the potassium salt compound represented by formula A-K is a crystalline hydrate, which has characteristic peaks at 2θ angles of 11.7±0.2°, 15.6±0.2°, 15.9±0.2°, 16.6±0.2°, 17.4±0.2°, 17.9±0.2°, 18.5±0.2, 21.4±0.2°, 23.5±0.2°, 24.0±0.2°, 27.7±0.2°, and 28.2±0.2° in the X-ray powder diffraction spectrum using Cu-Ka radiation;
more preferably, the crystalline hydrate of the compound represented by formula A-K has characteristic peaks at 2θ angles of 11.7±0.2°, 14.0±0.2°, 15.6±0.2°, 15.9±0.2°, 16.6±0.2°, 17.4±0.2°, 17.9±0.2°, 18.5±0.2°, 20.1±0.2°, 21.4±0.2°, 23.5±0.2°, 24.0±0.2°, 27.5±0.2°, 27.7±0.2°, 28.2±0.2°, 28.6±0.2°, 29.3±0.2°, and 29.6±0.2° in the X-ray powder diffraction spectrum using Cu-Ka radiation;
further preferably, the crystalline hydrate of the compound represented by formula A-K has an X-ray powder diffraction spectrum substantially as shown in FIG. 6;
preferably, the mass fraction of water in the crystal hydrate of the compound represented by formula A-K is 3.3-4.3%.
more preferably, the lithium salt compound represented by formula A-L is a crystalline hydrate, which has characteristic peaks at 2θ angles of 16.7±0.2°, 18.8±0.2°, 21.9±0.2°, and 23.9±0.2° in the X-ray powder diffraction spectrum using Cu-Ka radiation;
more preferably, the crystalline hydrate of the compound represented by formula A-L has characteristic peaks at 2θ angles of 5.6±0.2°, 8.8±0.2°, 11.8±0.2°, 14.0±0.2°, 16.3±0.2°, 16.7±0.2°, 16.9±0.2°, 17.0±0.2°, 17.7±0.2°, 18.5±0.2°, 18.8±0.2°, 21.9±0.2°, 23.9±0.2°, and 28.2±0.2° in the X-ray powder diffraction spectrum using Cu-Ka radiation;
further preferably, the crystalline hydrate of the compound represented by formula A-L has an X-ray powder diffraction spectrum substantially as shown in FIG. 8;
preferably, the mass fraction of water in the crystal hydrate of the compound represented by formula A-L is 3.6-4.6%.
